# EUROPEAN PATENT SPECIFICATION

(11) **EP 0 983 088 B1**
(45) Date of publication and mention of the grant of the patent: **25.10.2006**
(21) Application number: 98919633.2
(22) Date of filing: 18.05.1998
(51) Int. Cl.: A61K 39/39, A61K 9/00

(54) **Immunpotentiating composition in a solid dosage form, having collagen or polydimethylsiloxane as a carrier substance**
Immunverstärkende Zusammensetzung in fester Verabreichungsform mit Kollagen oder Polydimethylsiloxan als Trägersubstanzen
Composition immunostimulante sous forme d'administration solide et ayant comme substance porteuse le collagène ou la polydimethylsiloxane

(30) Priority: 19.05.1997 JP 14592097; 30.05.1997 JP 14246197; 30.10.1997 JP 31628597
(43) Date of publication of application: 08.03.2000
(73) Proprietor: Dainippon Sumitomo Pharma Co., Ltd., Osaka-fu (JP); The University of Melbourne, Parkville, VIC 3052 (AU); Koken Company Limited, Tokyo 161-0033 (JP)
(72) Inventor: FUJIOKA, Keiji, Toyonaka-shi, Osaka 560-0002 (JP); SANO, Akihiko, Toyonaka-shi, Osaka 560-0011 (JP); NAGAHARA, Shunji, Higashiosaka-shi, Osaka 577-0843 (JP); BRANDON, Malcolm, Roy, Ivanhoe East, Melbourne, VIC 3079 (AU); NASH, Andrew, Donald, Northcote, Melbourne, VIC 3070 (AU); LOFTHOUSE, Shari, Northcote, Melbourne, VIC 3070 (AU)
(74) Representative: Vossius & Partner
(86) International application number: PCT/JP1998/002172
(87) International publication number: WO 1998/052605

(56) References cited:
- EP-A- 0 659 406
- WO-A-91/01143
- WO-A-94/27718
- WO-A-95/31187
- WO-A-97/04012
- WO-A1-93/14785
- J.H. ELDRIDGE ET AL.: "BIODEGRADABLE AND BIOCOMPATIBLE POLY(DL-LACTIDE-Co-GLYCOLIDE) MICROSPHERES AS AN ADJUVANT FOR STAPHYLOCOCCAL ENTEROTOXIN B TOXOID WHICH ENHANCES THE LEVEL OF TOXIN-NEUTRALIZING ANTIBODIES." INFECTION AND IMMUNITY, vol. 59, no. 9, September 1991, pages 2978-2986, XP002073740 WASHINGTON US
- ANONYMUS: "Collagen corneal shields" UNIVERSITY OF ILLINOIS EYE CENTER Retrieved from the Internet: URL:http://www.uic.edu/com/eye/LearningAbo utVision/EyeFacts/CollagenCornealShields.s html> [retrieved on 2005-09-30] * the whole document *
- NAKAOKA ET AL: 'Size effect on the antibody production induced by biodegradable microspheres containing antigen' VACCINE vol. 14, no. 13, 1996, pages 1251 - 156
- CROTTS; PARK: 'Stability and release of bovine serum albumin encapsulated with poly(D,L-lactide-co-glycolide) microparticles' JOURNAL OF CONTROLLED RELEASE vol. 44, 1997, pages 123 - 134
- ROITT I.M. ET AL: 'Immunology', 1989, GOWER MEDICAL PUBLISHING, LONDON * page 11 - page 15 *

## Description

### Technical Field

The present invention relates to an immunopotentiating composition for effectively increasing an immune response derived from an antigen. The immunopotentiating composition according to the present invention is used primarily as a vaccine preparation in the field of human medicine or veterinary medicine for the purpose of preventing or treating diseases in human beings, in mammals other than human beings, and in birds. Furthermore, the immunopotentiating composition according to the present invention is used for immunizing animals for the purpose of antibody production.

### Background Art

Those vaccines that are currently in general use are classifiable roughly into two groups, attenuated (live) vaccines and inactivated (killed) vaccines. Attenuated vaccines are advantageous in that good immune responses can be generally obtained but are disadvantageous in that, from the safety viewpoint, there are such anxiety factors as toxicity restoration and adverse effects with them. Inactivated vaccines are safer as compared with attenuated vaccines but are disadvantageous in that single administration thereof can hardly produce a sufficient immunizing effect. In fact, in preventive vaccination with inactivated vaccines, two or three administrations are made at intervals of two to three weeks so that a satisfactory effect can be obtained.

Meanwhile, recent advances in the methodology of molecular biology have made it possible to identify disease-specific antigens useful in the prevention or treatment of diseases and produce synthetic antigens (component vaccines) mimicking the antigens identified by using the chemical or recombinant DNA technology. The thus-synthesised antigens are superior in purity, stability, specificity and safety to the conventional vaccinal antigens. From the practical viewpoint, however, they are generally low in antigenicity and this is the greatest problem to be solved. The advent of a method of effectively producing an antibody to an antigen having low antigenicity is thus earnestly desired from the standpoint of human and veterinary medicine.

Furthermore, when an inactivated vaccine or component vaccine is used, it is necessary, for attaining an extent of antibody production which is effective in the prevention or treatment of a disease, to make 2 or 3 administrations at intervals of 2 to 3 weeks, preferably 4 weeks or longer. Therefore, a vaccine capable of producing a sufficient effect upon single administration (single shot vaccine) is earnestly demanded in human and veterinary medicine. In the field of veterinary medicine, its main advantages include 1) time reduction, 2) cost reduction and 3) improved compliance. In human medicine, too, the three advantages mentioned above are important and, among them, improved compliance is particularly important in developing countries where a plurality of administrations with intervals observed is virtually impossible.

The problem of weak antigenicity of inactivated vaccines and component vaccines can be solved, at an experimental level, by using an adjuvant. In practice, however, this solution has various problems such as adverse effects. Adjuvant technologies which use artificial substances includes two methods. One comprises dispersing an antigen on the surface of oil or lipid particles and the other comprises causing an antigen to be adsorbed on a precipitate.

Mineral oil had been used for some time for veterinary vaccines or military influenza vaccines, causing, however, severe haemorrhagic lesions or protracted granuloma. Since then, common use thereof in vaccines for human use has never been approved by the relevant authorities. Freund's complete adjuvant and incomplete adjuvant have been used for the past four decades most widely in animal experiments. Though they induce antibody production to a satisfactory extent, however, they promote granuloma formation and adhesion at the site of infusion, pyrexia and other toxic effects and, accordingly, the use thereof in human or veterinary medicine has been avoided.

Alum, aluminum hydroxide or aluminum phosphate, is currently the only adjuvant approved for administration to humans and is in wide use. However, it causes granuloma formation at the site of vaccination and, in addition, its effect disadvantageously varies widely from case to case. For example, aluminum hydroxide produces a sufficient adjuvant effect with bacterial toxoids but, with vaccines against hepatitis B virus or influenza virus, no good results have been obtained.

In addition to the above-mentioned adjuvant techniques which use artificial substances, there is a method which uses, as an adjuvant, a cytokine originally occurring in the living body and having immunoactivating activity, in fact, it is reported or disclosed that antibody production in response to antigens is enhanced by the use of cytokines having immunoactivating activity (e.g. IL-1, IL-2, IFN-γ, IFN-α, GM-CSF, IL-12, etc.). These findings are reviewed, for example, by Rong Lin et al. (Clinical Infection Diseases, 21, 1439-1449, 1995). However, when a cytokine is used as an adjuvant in a dissolved state, a plurality of administrations are necessary to achieve a satisfactory antibody producing effect, although its adverse effects are scarce and less severe as compared with the artificial adjuvants mentioned above. It is supposed that as an organism is inoculated with an antigen and a cytokine in a dissolved state, they diffuse in the organism immediately after inoculation, thus failing to activate the immunomechanism specific to the antigen. Furthermore, for systemic immunoactivation with a cytokine, the cytokine is required in large quantities and, in that case, severe adverse effects may possibly be induced. Therefore, a method of using a cytokine effectively as an adjuvant without causing adverse effects is desired.

As a further approach to the solution of the weak antigenicity problem which inactivated vaccines and component vaccines have, there may be mentioned the technique of sustained release of the antigen from the carrier. The idea of sustained antigen release originates from the thinking that the adjuvant effect obtained with alum is due to nonspecific adsorption of the antigen on alum and sustained release thereof from alum. So far attempts have been made using various carriers (e.g. Bongkee Sah et al., J. Pharm. Pharmacol., 48, 32-36, 1996) but none has resulted in practical use.

The time period from antigen administration to antibody production is also very important from the viewpoint of disease prevention or treatment. No attempts have so far been made to curtail this period required for antibody production, however.

WO 97/04012 discloses a polysaccharide gel composition enclosing a biologically active substance for use as a sustained release composition or a depot composition. WO 93/14785 discloses a composition for immunotherapy against ocular Herpes simplex virus (HSV) infection using a collagen shield soaked in a concentrated solution containing HSV glycoprotein and an adjuvant, and its application to the eye. WO 94/27718 discloses a method of immunizing a mammal against a disease using antigen containing microparticles.

In view of the foregoing, the object of the present invention is:
(1) To provide an immunopotentiating composition which comprises an antigen or antigen-inducing substance and a carrier, wherein
   (a) the carrier is collagen or polydimethylsiloxane and the antigen or antigen-inducing substance is dispersed therein or encapsulated therewithin;
   (b) the antigen induces an immune response specific thereto, wherein the immune response is
      (i) an antibody production, effective in the prevention and/or treatment of diseases in a human, a mammal other than a human, or a bird,
      (ii) effective in the prevention or treatment of a disease,
      (iii) effective in the prevention of infection with a virus, mycoplasmata, bacterium or parasite, or
      (iv) used for animal production,
      or the antigen is a superantigen;
   (c) the immunopotentiating composition is in a solid dosage form; and
   (d) the antigen-inducing substance is a plasmid or virus with a nucleic acid (gene sequence) coding for an antigen capable of inducing an immune response specific to a member of the group consisting of viruses, mycoplasmata, bacteria, parasites, toxins and tumor cells as incorporated therein so that said antigen can be produced in vivo, or for a superantigen as incorporated therein so that said superantigen can be produced in vivo.

### Means for Solving the Problems

The present inventors made intensive investigations in an attempt to obtain a composition allowing sustained release of antigen from a solid biocompatible material which is collagen or polydimethylsiloxane and, as a result, unexpectedly found that when an immunopotentiating composition comprising a solid biocompatible material which is collagen or polydimethylsiloxane and an antigen carried thereon is administered to living organisms, the immune response derived from the antigen is enhanced.

Furthermore, the present inventors found that administration to the living body of the immunopotentiating composition of the invention comprising a substance having immunoactivating, immunostimulating or immunomodulating activity (hereinafter collectively referred to as "immunomodulating substance") as simultaneously borne on a carrier comprising a biocompatible material together with an antigen results in the production of an early and further enhanced immune response. Based on these findings, the present invention has now been completed.

In the following, the present invention is described in further detail.

### i) Explanation of the principle of the present invention

In typical humoral immune responses, the antibody production following the second stimulation with an antigen occurs earlier to a higher antibody titre, which is maintained for a longer period, as compared with the antibody production following the first stimulation with the antigen. The difference in the period required for antibody production is roughly due to the fact that while the first antigenic stimulation is to be followed by a series of steps, namely (1) presentation of the antigen to T cells by antigen presenting cells and activation of T cells, (2) activation of B cells by activated T cells, (3) transportation of the antigen to lymph nodes by dendritic cells and (4) proliferation of B cells in the lymph nodes and differentiation thereof into antibody forming cells, a sufficient number of antibody forming cells are already available at the time of second stimulation. The difference in antibody titre level and in duration of high antibody titres is due to the fact that, in immunologic stimulation using the conventional solution form, the antigen administered for the first immunologic stimulation diffuses throughout the body, degraded, metabolised and eliminated. The antigen has mostly disappeared from the body when antibody forming cells are prepared for antibody production; therefore, there is no stimulation by the antigen to the antibody forming cells again. For attaining a higher antibody titre of longer duration, which is important for the prevention or treatment of a disease, it is therefore necessary for the antibody forming cells produced in response to the first antigenic stimulation to be stimulated again by the antigen.

On the other hand, earlier antibody production is also important in the prevention or treatment of a disease. For earlier antibody production, it is important to cause efficient production of antibody forming cells by the first antigenic stimulation. For such efficient antibody forming cell production, it is necessary to (1) increase the chances of contact of the antigen with antigen presenting cells (causing antigen presenting cells to accumulate at the site of administration of the antigen) and (2) enhance the activation of B cells and the differentiation thereof into antibody forming cells in lymph nodes.

With these points in view, the present invention realised higher antibody titres of longer duration by causing an antigen to be stably borne on a carrier comprising a biocompatible material and be released sustainedly therefrom to thereby maintain the antigen amount in the body, causing the antigen to stimulate again the antibody forming cells produced. In particular, while it is readily estimable that the antigen concentration be maintained at a high level at and around the site of administration of the immunopotentiating composition, this local high antigen concentration state promotes the reaction between the antigen and antibody forming cells, which is an equilibrium reaction, and at the same time causes accumulation of immunocompetent cells at and around the site of administration. Therefore, to maintain the local antigen concentration at a high level may be mentioned as the most important principle of the present invention.

Furthermore, the present invention realised earlier and more efficient antibody production by causing an antigen and an immunomodulating substance (for instance cytokine) to be simultaneously borne on a carrier comprising a biocompatible material and be released sustainedly therefrom to thereby (1) promote the local accumulation of immunocompetent cells at and around the site of administration of the antigen and the subsequent activation of antigen presentation to T cells and (2) enhance the activation of B cells and the differentiation thereof into antibody forming cells in the lymph node in charge of the site of administration of the immunopotentiating composition (that lymph node to which dendritic cells transfer the antigen and in which antibody forming cells are produced) through selective and continued inflow of the cytokine into said lymph node. Therefore, a characteristic feature of the immunopotentiating composition according to the present invention consists in that, unlike the systemic immunoactivating mechanism induced by administration of an antigen, or an antigen and a cytokine, in the form of a solution, a field for immunopotentiation is formed around the composition through sustained release of the antigen, or the antigen and the cytokine.

In view of the foregoing, the effects of the present invention may be summarised as follows:
(1) The antigen or antigen-inducing substance (hereinafter collectively referred to as "antigenic substance") and, when present, the immunomodulating substance may be released sustainedly.
(2) The concentration of the antigenic substance and, when present, the immunomodulating substance may be maintained at a high level or levels at the site of administration.

These features may be provided by stably maintaining, in the living organism, the antigenic substance or the antigenic substance and immunomodulating substance, which is/are borne or supported on a solid carrier constituting the immunopotentiating composition of the invention and allowing said substance(s) to be sustainedly released in said organism. Since the immunopotentiating composition is administered to living organisms, it is of course required that the carrier should be a material having good biocompatibility.

Furthermore, these fundamental characteristics of the present invention can be said not only in regard to humoral immunity but also in regard to mucosal immunity and cell-mediated immunity since, in each case, the immunity is activated as a result of continuous antigenic stimulation of immunocompetent cells and positive acceleration of T cell and B cell activation.

### ii) Effects of the present invention

The effects produced by the present invention are mentioned below.

### a) Sustained release of an antigen or of an antigen and a cytokine

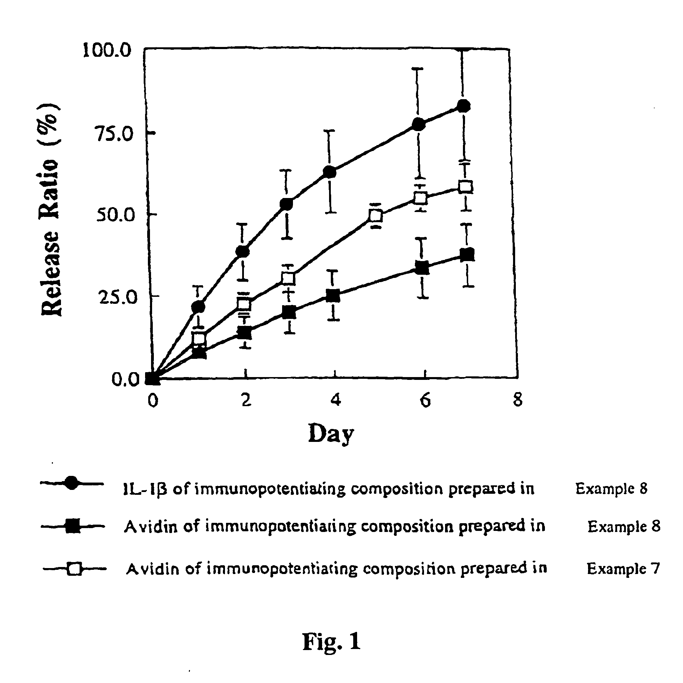

As shown in Fig. 1, an immunopotentiating composition of the present invention released an antigen (avidin) and a cytokine (IL-1β) sustainedly over 7 days or longer.

### b) Enhancement of antibody production

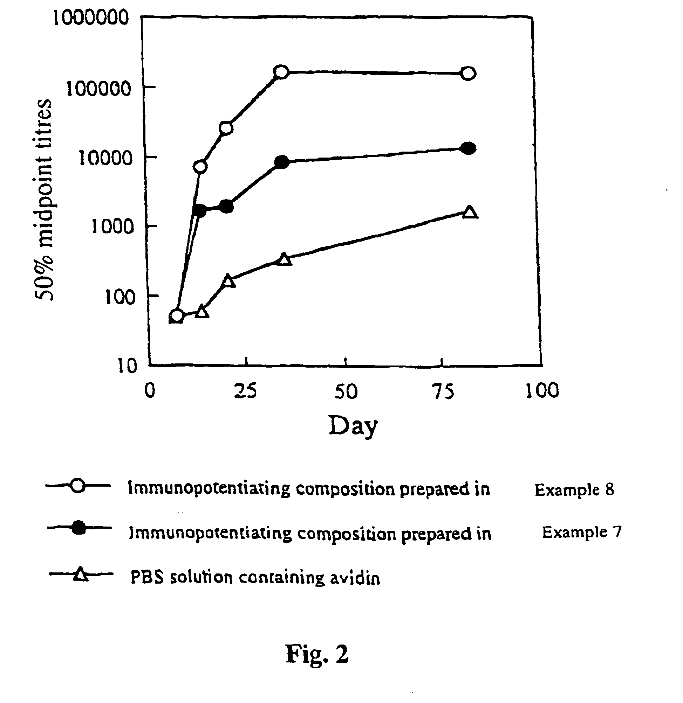

The antibody production enhancing effect of the immunopotentiating composition of the present invention was established in immunologic stimulation experiments in mice and sheep. Thus, avidin was administered, in varied dosage forms, to mice and anti-avidin antibody titres in blood were determined by the ELISA technique at 7, 14, 21, 35 and 83 days after administration (Fig. 2). The case in which 100 micrograms of avidin was administered to mice in the conventional manner, namely in the form of a solution of avidin in phosphate buffer, the case in which an immunopotentiating composition (prepared in Example 7) carrying the same amount of avidin was administered, and the case in which an immunopotentiating composition (prepared in Example 8) carrying the same amount of avidin simultaneously with IL-1β was administered were compared with one another. At 35 days after administration, the antibody titre in blood of the mice given the avidin-carrying immunopotentiating composition was about 25 times higher as compared with the antibody titre obtained in the mice given the avidin solution. This result indicates that the use of the antigen in the immunopotentiating composition form according to the present invention resulted in enhanced antibody production in response to the antigen. Furthermore, in the mice given the immunopotentiating composition carrying avidin and IL-1β, the antibody titre was as high as about 450 times the antibody titre attained upon administration of the avidin solution. This result indicates that the antibody production in response to an antigen can be further enhanced by using the immunopotentiating composition of the present invention which simultaneously contains an antigen and a cytokine having immunoactivating activity.

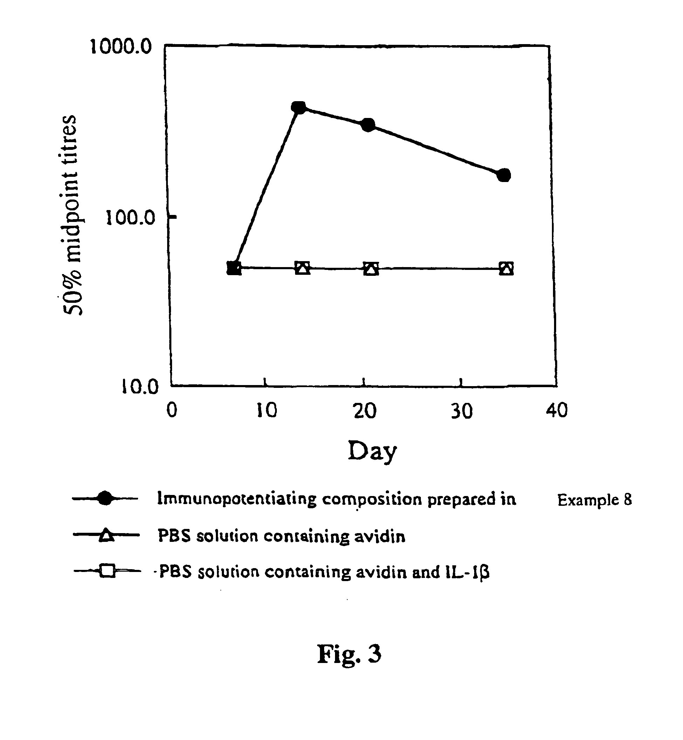

The antibody producing effect of the immunopotentiating composition carrying an antigen and an immunoactivating cytokine simultaneously was more markedly observed in an immunostimulation experiment in sheep (Fig. 3). Avidin was administered in varied dosage forms to sheep and anti-avidin antibody titres in blood were determined by the ELISA technique at 7, 14, 21 and 35 days after administration. When avidin was administered in the conventional solution form, no anti-avidin antibody production was confirmed even at a dose of 100 micrograms. This difference in antibody production between mice and sheep is supposedly due to the difference in body weight. This indicates that 100 micrograms of avidin has no sufficient antigenicity to cause antibody production in sheep. In contrast, when an immunopotentiating composition (prepared in Example 8) carrying avidin and IL-1β simultaneously was administered, a high level of antibody production was established at 14 days after administration.

This result clearly indicates that the immunopotentiating composition of the present invention has an enhancing effect on the antibody production in response to an antigen having only insufficient antigenicity to cause antibody production. In contrast, when avidin and IL-1 β were simultaneously administered in the conventional solution form, no antibody production was detected. This result indicates that the antibody production enhancing effect produced by the immunopotentiating composition depends on the sustained release of the antigen and cytokine. The above finding indicates that in contrast to the conventional method of administration which requires a plurality of administrations for attaining a sufficient antibody titre, the immunopotentiating composition of the present invention can give a sufficient antibody titre after only one administration.

### c) Reduction in time until antibody production

The reduction in time until antibody production, which is one of the important effects of the immunopotentiating composition of the present invention, was directly ascertained in the immunostimulating experiment using mice, in which even upon administration of the antigen solution in the conventional manner, a certain extent of antibody production was observed, rather in the immunostimulating experiment using sheep in which the administration of the antigen solution in the conventional manner failed to lead to antibody production. As is clear from the graphic representation in Fig. 2, when avidin was administered in solution form in the conventional manner, the anti-avidin antibody titre increased from the 14th day after administration, whereas when the immunopotentiating composition (carrying avidin alone or avidin plus IL-1β) was administered, the antibody amount showed a rapid increase from the 7th day after administration, whereby the time until antibody production was shortened by about a week. It took 83 days after administration for the antibody titre in mice given avidin in solution form to arrive at the same antibody titre as that obtained on the 14th day after administration of the immunopotentiating composition carrying avidin alone. With the avidin solution, the antibody titre obtained on the 14th day after administration of the immunopotentiating composition carrying avidin and IL-1β could not be attained even on the 83rd day after administration. In this respect, it may be said that the immunopotentiating composition curtailed the period required for antibody production by at least 69 days.

### d) Immune response in the neighbourhood of the site of administration of the immunopotentiating composition

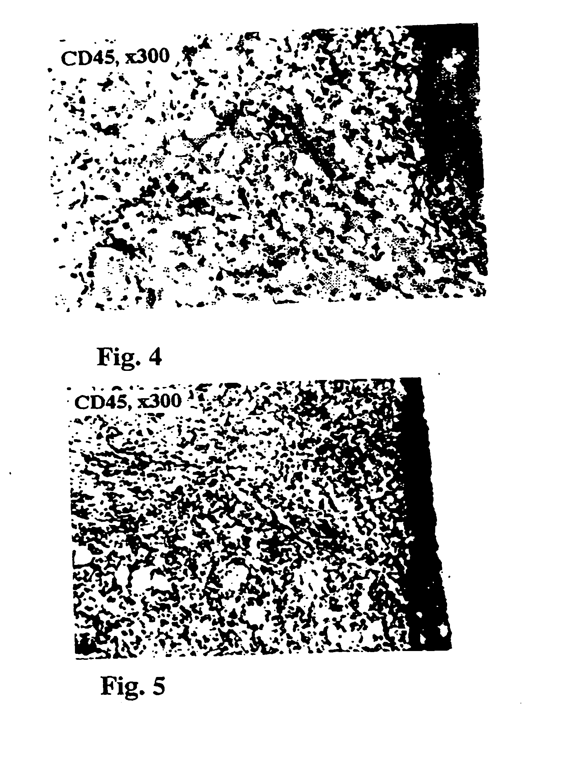

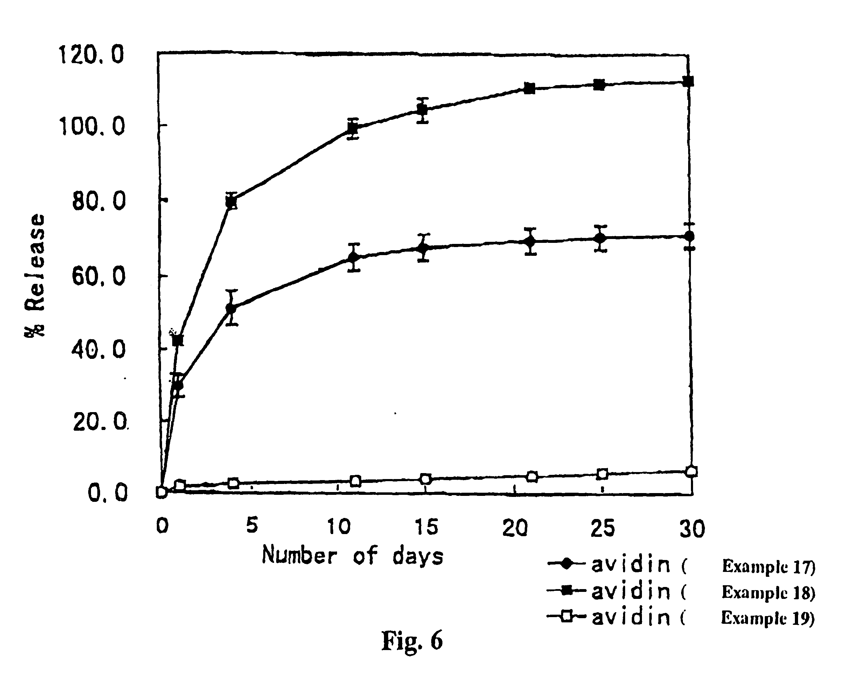

As already mentioned in the section "explanation of the principle of the present invention", the immune response occurring locally in the neighbourhood of the site of administration seems to play an important role in the effect obtainable with the immunopotentiating composition of the present invention. This could be easily ascertained from the histological picture at the administration site of the sheep used in the immunostimulation experiment (Fig. 4, 5). The tissue photomicrograph shows infiltration of immunocompetent cells around the immunopotentiating composition. The immunocompetent cells as so called herein include neutrophils, CD4-positive T cells, y δ TCR-positive T cells, MHC II-positive cells, macrophages and so forth.

Such accumulation of immunocompetent cells was not observed when avidin and/or IL-1 β was administered in a solution form. This is presumably due to immediate diffusion in the body, following inoculation, of the avidin and IL-1β in solution form, resulting in failure to marshal immunocompetent cells to the site of inoculation. The accumulation of immunocompetent cells was more conspicuous with the immunopotentiating composition carrying IL-1β. These findings are supportive of the conception that a state of high antigen, or antigen and cytokine concentration is created around the immunopotentiating composition as a result of sustained release from said composition, resulting in antibody production enhancement through accumulation of immunocompetent cells around said composition.

On the other hand, accumulation of immunocompetent cells is a sort of inflammatory response. However, the inflammatory response evoked was not accompanied by oedema or the like but ceased by itself.

### iii) The immunopotentiating composition

The term "immunopotentiating composition" as used herein means, in principle, a composition or preparation comprising a solid carrier, which is biocompatible collagen or polydimethylsiloxane material, and an antigen or antigen-inducing substance borne on said carrier and, if desired, further comprising an immunomodulating substance, as described below, and/or one or more pharmaceutical additives.

The "immune response" to be potentiated by the immunopotentiating composition of the present invention is the immune response specific to the antigen contained in said composition or the antigen induced by the inducer contained therein. The immune response to be activated may be humoral immunity, mucosal immunity or cellular immunity, or a combination thereof.

The term "antigen" is not limited to any particular species provided that it car induce the antigen-antibody reaction derived from the antigen. Generally, it is selected from among those antigens to which antibodies effective in the prevention and/or treatment of diseases in humans or mammals other than humans or in birds are produced. Thus, it includes, but is not limited to, those toxoids, vaccines and live vaccines themselves that are described, for example, in "Vaccine Handbook" (edited by the National Institute of Health Alumni Association, published by Maruzen Co.), "Immunizing Agents" in Remington's Pharmaceutical Sciences, 14th edition, 1990, Mack Publishing Co., Section 75, pages 1426-1441 or Physician's Desk Reference to drugs approved by the United States Food and Drug Administration, 46th edition, pages 208-209, 1992. Furthermore, it includes, but is not limited to, the following:
(1) Viruses, mycoplasmata, bacteria, parasites, toxins, tumor cells and the like attenuated or rendered non-toxic or non-pathogenic, for example by gene recombination (modification of the toxicity- or pathogenicity-related gene), continued subculturing (appearance of attenuated or non-pathogenic strains as a result of self-modification), formalin treatment, β -propiolactone treatment, exposure to radiation, sonication, enzyme treatment, heating or the like.
(2) Proteins such as membrane surface proteins and nuclear proteins, proteoglycans, polypeptides, peptides, membrane components and the like obtained from viruses, mycoplasmata, bacteria, parasites, toxins, tumor cells and the like, for example by chemical or enzymatic degradation, physical disruption, column purification, extraction or filtration.
(3) Subunit vaccines, synthetic peptides having a sequence such that they are comparable or superior in specific antigenicity to the corresponding antigens, and the like, as obtained by excising a gene coding for an antigen capable of inducing specific immunity to a virus, mycoplasma, bacterium, parasite, toxins, tumor cell line or the like from the corresponding virus, mycoplasma, bacterium, parasite, tumor cell line or the like, identifying said gene, inserting it into an appropriate vector such as a plasmid, and causing the gene to be expressed in Escherichia coli, yeasts or animal cells. The antigen capable of inducing an immune response specific to tumor cells, so referred to herein, includes, but is not limited to, the so-called tumor regression antigens such as MAGE-1, MAGE-3 and BAGE, tissue-specific antigens such as tyrosinase, Mart-1, gp100 and gp75, and, further, p15, Mucl, CEA, HPV E6, E7, HPR2/neu, and the like.

The "antigen" includes, but is not limited to, antigens capable of inducing an immune responses responsible for the onset of, or effective in the treatment of, such a disease as mentioned below: cholera, pertussis, plague, typhoid fever, meningitis, pneumonia, leprosy, gonorrhoea, dysentery, polio, gram-negative sepsis, colibacillemia, rabies, diphtheria, botulism, tetanus, poliomyelitis, influenza, Japanese encephalitis, rubella, measles, yellow fever, parotiditis, hepatitis A, hepatitis B, hepatitis C, varicella/herpes zoster, malaria, tuberculosis, candidiasis, dental caries, acquired immunodeficiency syndrome, cancer (tumor), matitis, anthrax, brucellosis, caseous lymphadenitis, enterotoxemia, enteritidis, black disease, malignant oedema, black leg, leptospirosis, scabby mouth, vibriosis, erysipelas, strangles, bordetella bronchitis, distemper, panleucopenia, rhinotracheit, viral diarrhoea and pimelea poisoning.

The antigen further includes, but is not limited to, antigens capable of inducing an immune response effective in the prevention of infection with such a virus, mycoplasma, bacterium or parasite as mentioned below, in the prevention of the onset of the relevant disease and in the treatment of patients with such disease: Pseudomonas aeruginosa, Staphylococcus aureus, Staphylococcus epidermidis, salmonellae, group B meningococci, group B streptococci, adenovirus, coronavirus, RS virus, human immunodeficiency virus I and II, herpes simplex I and II, CMV, EBV, Chlamydia trachomatis, parvovirus, parainfluenza virus, calicivirus.

The "antigen" also includes antigen which is not used for only animal health but also animal production. For example, the antigen is described in "Vaccines in Agriculture, Immunological Applications to Animal Health and Production" (edited by P. R. Wood et al., CSIRO, 71-160, 1994). The antigen includes, but in not limited to, antigens used for animal production as mentioned below: 1)antigens for reproduction; antigens which can induce immunoresponse against inhibin related peptides and releasing hormones, such as luteinising hormone releasing hormone, gonadotrophin releasing hormone, etc.: 2)antigens for control of growth and metabolism of animal; antigens which can induce immunoresponse against growth hormone relating factor, insulin-like growth factor-1, growth hormone, steroid hormones, sex steroid hormones, plasma membrane antigens of adipocyte, fat lipids, cortisol, adenocorticotrophic hormone, adenocorticotrophic hormone receptor, β -adrenergic receptor, adenohypophyseal hormones, such as prolactin, ACTH, STH, TSH, LH, FSH, etc.,: 3)antigens for environmental control; antigens which can induce immunoresponse against plant-associated toxins, low molecular weight natural toxicants.

Further, the term "antigen" is not limited to any particular species provided that it can induce specific immune response to antigens and also includes antigens capable of inducing non-specific immune response to antigens. The antigen capable of inducing non-specific immune response to antigen, so referred to herein, includes, but is not limited to, the so-called superantigens such as staphylococcal enterotoxins, toxic shock syndrome toxin-1, exofoliative dermatitis toxin, CAP (cell-membrane associated protein) and SPM (Streptococcus pyogenes-mitogen) such as T-12 and NY-5 described in Miyagiken Ishikai Kaiho, Vol. 50, 133-137, 1997, and the like.

The term "antigen-inducing substance" means a substance capable of inducing such an antigen as mentioned above in vivo and includes, among others, plasmids and viruses containing a nucleic acid encoding a gene sequence for an antigen capable of inducing specific immunity to a virus, mycoplasma, bacterium, parasite, toxins, tumor cells or the like as inserted therein so that the relevant antigen can be produced in vivo.

The nucleic acid to be inserted includes, but is not limited to, nucleic acids coding for sucstances capable of serving as antigens such as mentioned above, for example nucleic acids coding for the following proteins: the influenza HA or NA, or NP protein, the type C hepatitis virus E2 or NS 1 protein, the type B hepatitis virus HBs antigen protein, the type A hepatitis virus capsid protein VP1 or VP3, capsidoid proteins, the dengue virus Egp protein, the RS virus F or G protein, the rabies virus G or N structural protein, the herpes virus gD protein, the Japanese encephalitis virus E1 or pre-M protein, the rotavirus coat protein VP7 or coat protein VP4, the human immunodeficiency virus gp120 or gp160 protein, the Leishmania major surface antigen protein, the malaria circum sporozoite major surface antigen protein, the Toxoplasma 54-kd or CS protein, the cell furface protein PAc of caries-causing Streptococcus mutans, such tumor regression antigens as MAGE-1, MAGE-3, and BAGE, such tissue-specific antigens as tyrosinase, Mart-1, gp100 and gp75, nucleic acids coding for p15, Muc1, CEA, HPV, E6, E7, HPR2/neu, etc., and those nucleic acids which are described in "Immunization with DNA"; Journal of Immunological Methods, vol. 176, 1994, pages 145-152.

The plasmids or viruses into which such nucleic acid is to be inserted are not limited to any particular species provided that they are non-pathogenic. Thus, as the viruses, there may be mentioned those viruses that are generally used as vectors in gene therapy, for example adenoviruses, adeno-associated viruses, vaccinia viruses, retroviruses, HIV viruses and herpes viruses.

The antigenic substance can be obtained by using the chemical, recombinant DNA, cell culture or fermentation technology. In the practice of the present invention, the method of preparing said substance is not limited to any specific one. Since, however, the composition of the present invention has the effects mentioned above, those antigens are particularly suited for use which are obtained by the recombinant DNA technology, are thus low in antigenicity and, in general, can hardly be produced in an efficient manner following administration by the conventional method (e.g. parenteral administration in the state of solution or suspension).

The antigenic substance for inducing specific immunity may be incorporated as such in the immunopotentiating composition, without any modification or, for further increasing its antigenicity and/or increasing its stability, it may be, for instance, (1) bound either covalently or non-covaiently to a protein having a higher molecular weight than the antigen, for example β-galactosidase or a core protein, (2) supplemented with an appropriate sugar (carbohydrate) chain, (3) included in liposomes, (4) included in virus-liposome membrane fusion type liposomes or (5) contained in virosomes obtained by using B30MDP [6-0-(2-tetradecyl hexadecanoyl)-N-acetylmuramyl-L-alanyl-D-isoglutamine].

The "immunomodulating substance (substance having immunoactivating, immunostimulating or immunomodulating activity)" is not limited to any particular species but includes, among others, cytokines, chemokines, growth factors, adjuvant peptides and DNA sequences, alum, Freund's complete adjuvant, Freund's incomplete adjuvant, iscom, saponins, hexadecylamine, dimethyldioctadecylammonium bromide, Abridin, cell wall skeletal components, cholera toxin, lipopolysaccharide endotoxins, liposomes including cytokine-containing liposomes and Walter Reed liposomes, 1,25-dihydroxyvitamin D3, and gelation products from a carboxylvinyl polymer, alginin and sodium chloride. The "cytokine" is not limited to any particular species provided that it has immunoactivating activity, thus including, among others, IFN―α,IFN―β, IFN-γ, IL-1 α, IL-1 β, IL-2, IL-3, IL-4, IL-5, IL-6, IL-7, IL-8, IL-12, TNF-α, TNF-β and GM-CSF. For example, when accumulation of immunocompetent cells around the site of administration of the immunopotentiating composition and the subsequent enhancement of the antibody production are desired, IL-1β and IL-2 are particularly preferred.

Specific adjuvants of interest, include, but are not limited to one or more of the group selected from Adju-Phos, Algal Glucan, Algammulin, Alhydrogel, Antigen Formulation, Avridine, Bay R1005, Calcitriol, Calcium Phosphate Gel, Cholera Holotoxin (CT), Cholera Toxin B Subunit (CTB), CRL1005, DDA, DHEA, DMPC, DMPG, DOC/Alum Complex, Gamma Inulin, Gerbu Adjuvant, GMDP, Imiquimod, ImmTher, Interferon-gamma, ISCOM(s), Iscoprep 7.0.3, Loxoribine, LT-OA or LT Oral Adjuvant, MF59, MONTANIDE ISA 51, MONTANIDE ISA 720, MPL, MTP-PE, MTP-PE Liposomes, Murametide, Murapalmitine, D-Murapalmitine, NAGO, Nonionic Surfactant Vesicles, Pleuran, PLGA, PGA and PLA, Pluronic L121, PMMA, PODDS, Poly Ra: Poly rU, Polyphosphazene, Polysorbate 80, Protein Cochleates, QS-21, Quil A, Rehydragel HPA, Rehydragel LV, S-28463, SAF-1, Sclavo Peptide, Sendai Proteoliposomes, Sendai-Containing Lipid Matrices, Span 85, Specol, Squalane, Squalene, Stearyl Tyrosine, Theramide, Threonyl-MDP, Ty Particles.

The amount of the immunity inducing antigenic substance and that of the immunomodulating substance contained in the immunopotentiating composition of the present invention can be adjusted arbitrarily according to the mixing ratios to the biocompatible material and additive(s) contained in the composition and to the form or size of the composition. The dose of the antigenic substance to be administered by means of the composition of the present invention may be approximately the same as that employed for the conventional method of administration (e.g. parenteral administration in a solution or suspension form).

However, since the composition of the present invention has an excellent immunopotentiating effect, as mentioned above, immunity can sufficiently be induced at lower doses as compared with the conventional method of administration, and the dose can suitably be adjusted according to the antigenic substance, the dosage form of the composition of the present invention and/or the immunomodulating substance to be administered simultaneously with the antigenic substance and the amount thereof.

The form or shape of the solid immunopotentiating composition of the present invention may be rod- or bar-like or particle-like, for instance. A suitable form can be selected so that an immune response can be induced more efficiently. A rod-like shape is preferred. A coated or covered rod formulation such as described in EP 659,406 is more particularly preferred.

For example, a rod- or bar-like form can release the antigenic substance and, when present, immunomodulating substance over a prolonged period of time, while a particle-like composition can readily undergo phagocytosis by immunocompetent cells such as macrophages. In the case of particles or fine granules, the diameter thereof is desirably, but is not limited to, 0.1 micrometers to 100 micrometers, more desirably 0.5 micrometers to 50 micrometers.

The biocompatible carrier according to the present invention may be such that the antigenic substance is dispersed therein or encapsulated therewithin. The biocompatible carrier may be such that it provides delayed and/or sustained release of the antigenic substance.

The biocompatible carrier may be collagen or polydimethylsiloxane. The biocompatible material meets the condition that it does not denature and/or inactivate the antigenic substance, or the antigenic substance and the immunomodulating substance, in the process for preparing the immunopotentiating composition.

As particularly preferred biodegradable and biocompatible materials, there may be mentioned collagen. Any collagen species can be used provided that it is suited for the purpose of the present invention. Thus, use may be made of animal- or plant-derived acid-soluble collagens, salt-soluble collagens, and alkali-soluble collagens, derivatives of these such as atherocollagens, side chain-modified collagens and crosslinked collagens, and genetically engineered collagens, preferably atherocollagens, side chain-modified collagens and cross-linked collagens. As the side chain-modified collagens, there may be mentioned, for example, succinylated, methylated or myristylated collagens. As the cross-linked collagens, there may be mentioned, for instance, glutaraldehyde-, hexamethylene diisocyanate- or polyepoxy compound-treated collagens (Fragrance Journal, 1989 (12), 104-109; Japanese Patent Publication (Kokoku) 07-59522).

Polydimethylsiloxane may be mentioned as a particularly preferred non-biodegradable biocompatible material and it is also desirable that one or more of the biocompatible materials mentioned above be used in admixture with this polydimethylsiloxane. Said polydimethylsiloxane is not limited to any particular species but, from the ease of moldability and other viewpoints, such silicones as Silastic (registered trademark) medical grade ETR elastomer Q7-4750 and Dow Corning (registered trademark) MDX-4-4210 medical grade elastomer are particularly preferred.

For stabilisation of the antigenic substance, or the antigenic substance and the immunomodulating substance and/or controlling the release thereof, one or more pharmaceutical additives may be added. The pharmaceutical additives include, but are not limited to, albumin, glycine, amino acids other than glycine, polyamino acids, gelatin, chondroitin sulfate, sodium chloride, mannan, glucomannan, tannic acid, sodium citrate, mannitol and so forth.

The inventors have found that, in this form, the antigenic substance may be stable at room temperature and thus does not require cold storage. Further the immunomodulating agent may be introduced into the immunopotentiating composition directly; that is, a solvent is not required.

The method of administering the immunopotentiating composition of the present invention is not particularly limited but includes parenteral administration, oral administration, administration into the nasal cavity and/or lungs, shooting using compressed air, and retention or embedding at the site of incision. A desirable method of administration can be selected according to the form of the immunopotentiating composition in a manner such that an immune response can be induced more efficiently. In the case of rod- or bar-like compositions, parenteral administration or retention at the site of incision is desirable while, in the case of particles, they may be applied as such directly to the site of incision for retention, or may be parenterally administered in the form of a suspension prepared by suspending them in a solvent for injection, as described in Japanese Patent Publication (Kokoku) 03-72046 or, further, may be administered by shooting by means of compressed air using the Helios Gene Gun System (Bio-Rad) or a powder gun described in Proc. Natl. Acad. Sci. USA, 93, 6291-6296 (1996), for instance.

Although solvent for injection should be selected depending on properties of biocompatible material, antigenic substance and immunomodulating substance, the term "solvent for injection" is not limited to any particular species provided that 1) particle can be dispersed to the solvent, 2) particle can maintain its form when the particle is dispersed to the solvent, 3) antigenic substance and immunomodulating substance can be held in the particle when the particle is dispersed to the solvent, 4) the solvent is non-toxic, 5) the solvent in which particle is dispersed is non-toxic. For example, solvent for injection, so referred to herein, includes, but is not limited to, distilled water, physiological saline, phosphate buffered solution, soybean oil, sesame oil, peanut oil, cotton seed oil, MCT (medium-chain fatty acid triglycerides), olive oil, corn oil, castor oil, silicone oils, PEG (polyethylene glycol), PG (propylene glycol), and fatty acids used in preparing liposomes, such as DOTMA, DOPE, DOGS, etc.

As the method of producing the immunopotentiating compositions in biodegradable solution, suspension and hydrous gel form, there may be mentioned, for example, the method comprising allowing a solution, suspension or gel containing an antigenic substance, or an antigenic substance and an immunomodulating substance, to be added to a powder-form carrier containing, if necessary, one or more additives,

The method of producing the immunopotentiating composition in biodegradable solid form includes, but is not limited to, the method of Fujioka et al. (Japanese Patent Publication (Kokoku) 07-59522). As other methods, there may be mentioned
(1) the method comprising admixing an antigenic substance, or an antigenic substance and an immunomodulating substance, in powder, solution, suspension or gel form with a solution- or gel-form carrier containing, if necessary, one more additives, followed by drying,
(2) the method comprising admixing an antigenic substance, or an antigenic substance and an immunomodulating substance, in powder, solution, suspension or gel form with a powder-form carrier containing, if necessary, one or more additives, followed by drying,
(3) the method comprising allowing a solution, suspension or gel containing an antigenic substance, or an antigenic substance and an immunomodulating substance, to be added to a sponge-form carrier containing, if necessary, one or more additives, followed by drying,
(4) the method comprising allowing a solution, suspension or gel containing an antigenic substance, or an antigenic substance and an immunomodulating substance, to be added to a sponge-form carrier containing, if necessary, one or more additives, followed either by direct drying or by adding water or the like if necessary, kneading and drying,
(5) the method comprising milling the solid obtained in methods (1)-(4), followed by compression molding,
(6) the method comprising admixing an antigenic substance, or an antigenic substance and an immunomodulating substance, in powder form, with a powder-form carrier containing, if necessary, one or more additives, followed by compression molding.

The method of producing the immunopotentiating composition in the form of biodegradable particles includes, among others, but is not limited to,
(1) the method comprising spray-drying a solution containing an antigenic substance, or an antigenic substance and an immunomodulating substance and the carrier, if necessary together with one or more additives,
(2) the method comprising lyophilising a solution containing an antigenic substance, or an antigenic substance and an immunomodulating substance and the carrier, if necessary together with one or more additives, followed by grinding the sponge-like lyophilisate obtained, and
(3) the method comprising adding a solution containing an antigenic substance, or an antigenic substance and an immunomodulating substance and the carrier, if necessary together with one or more additives dropwise to a stirred solution in which the carrier is insoluble, and drying the particles obtained.

The method of drying, the temperature and humidity in the drying step, the method of mixing, the temperature and humidity in the mixing step, the method of compression molding, the temperature, humidity and molding pressure in the compression molding step, the viscosity of the carrier solution and of the active substance, or the antigenic substance and the immunomodulating substance solution, and the viscosity and pH of the carrier- antigenic substance mixed solution and of the antigenic substance- immunomodulating substance mixed solution may be the same as in the conventional methods.

The method of producing the immunopotentiating composition in non-biodegradable solid form includes, but is not limited to,
(1) the method comprising admixing an antigenic substance, or an antigenic substance and an immunomodulating substance, in powder, solution, suspension or gel form with a carrier monomer with one or more additives added thereto if necessary, adding a hardening agent, molding in an arbitrarily selected mold by filling or extrusion and effecting hardening,
(2) the method comprising admixing an antigenic substance, or an antigenic substance and an immunomodulating substance, in solution, suspension or gel form with a powder-form carrier containing, if necessary, one or more additives, followed by drying,
(3) the method comprising admixing an antigenic substance, or an antigenic substance and an immunomodulating substance, in powder, solution, suspension or gel form with a powder-form carrier containing, if necessary, one or more additives, and 1) filling the mixture into an arbitrarily selected mold, followed by compression molding, or 2) extruding the mixture using a nozzle,
(4) the method comprising admixing an antigenic substance, or an antigenic substance and an immunomodulating substance, in solution, suspension or gel form with a sponge-form carrier containing, if necessary, one or more additives, followed by drying,
(5) the method comprising admixing an active substance, or an active substance and immunomodulating substance, in powder, solution, suspension or gel form with a sponge-form carrier containing, if necessary, one or more additives, and 1) filling the mixture into an arbitrarily selected mold, followed by compression molding or 2) extruding the mixture using a nozzle,
(6) the method comprising forming, by the methods (1), (3) and (5), a rod or bar-like inner layer containing an antigenic substance, or an antigenic substance and an immunomodulating substance, and then coating the inner layer with an antigenic substance and an immunomodulating substance-free outer layer material, and
(7) the method comprising forming an inner layer and an outer layer simultaneously by coextrusion using a nozzle, among others. The method of drying, the temperature and humidity in the drying step, the method of mixing, the temperature and humidity in the mixing step, the method of compression molding, the temperature, humidity and molding pressure in the compression molding step, the viscosity of the carrier solution and of the antigenic substance, or the antigenic substance and the immunomodulating substance solution, and the viscosity and pH of the carrier-antigenic substance mixed solution and of the antigenic substance-immunomodulating substance mixed solution may be the same as in the conventional methods.

The method of using the immunopotentiating composition of the present invention is the use as an immunizing preparation to be administered to animals for the purpose of producing antibodies.

The present invention will now be more fully described with reference to the accompanying figures and examples. It should be understood, however, that the description following is illustrative only and should not be taken in any way as a restriction on the generality of the invention described above.

### Brief Description of the Drawings

**Figure 1**
   Time courses of cumulative releases of avidin and IL-1 β from the immunopotentiating compositions as found in Test Example 1 and Test Example 2.
**Figure 2**
   Time course of anti-avidin antibody titre in mice.
**Figure 3**
   Time course of anti-avidin antibody titre in sheep.
**Figure 4**
   Histological photomicrograph of the site of administration of the immunopotentiating composition prepared in Test Example 7 (CD45, x300).
**Figure 5**
   Histological photomicrograph of the site of administration of the immunopotentiating composition prepared in Test Example 8 (CD45, x300).
**Figure 6**
   Time courses of cumulative releases of avidin from the immunopotentiating compositions as determined in Test Example 5.
**Figure 7**
   Time courses of cumulative releases of avidin and IL-1 β from the immunopotentiating compositions as determined in Test Example 6.
**Figure 8**
   Time courses of anti-avidin antibody titres in mice as found in Test Example 7.
**Figure 9**
   Time courses of anti-avidin antibody titres in mice as found in Test Example 8.
**Figure 10**
   Time course of body temperature and white blood cell count of sheep after administration of silicone alone in Test Example 9.
**Figure 11**
   Time course of body temperature and white blood cell count of sheep after administration of silicone based immunopotentiating compositions containing IL-1β in Test Example 9.
**Figure 12**
   Time course of body temperature and white blood cell count of sheep after administration of silicone based immunopotentiating compositions containing IL-1β and avidin in Test Example 9.

### EXAMPLES

### (1) Preparation of immunopotentiating compositions

### Example 1

An aqueous solution (10 ml) containing 5.0 mg/ml of avidin (Boehringer Mannheim GmbH, Germany) and 3 ml of an aqueous solution containing 100 mg/ml of glycine (Nakalai Tesque, Inc., Japan) were admixed with 134 g of an atelocollagen solution (Koken Co., Ltd., Japan; atelocollagen content: 2%) to give an immunopotentiating composition in solution form.

### Example 2

An aqueous solution (1.7 ml) containing 5.0 mg/ml of sheep IL-1β (prepared by the method of A. E. Andrews et al., Vaccine, 12, 14-22, 1994), 8.6 ml of an aqueous solution containing 5.0 mg/ml of avidin and 1.5 ml of an aqueous solution containing 100 mg/ml of glycine were admixed with 142 g of the 2% atelocollagen solution to give an immunopotentiating composition in solution form.

### Example 3

A sponge-form immunopotentiating composition was obtained by lyophilising the solution-form immunopotentiating composition prepared in Example 1.

### Example 4

A sponge-form immunopotentiating composition was obtained by lyophilising the solution-form immunopotentiating composition prepared in Example 2.

### Example 5

A gel-form immunopotentiating composition was obtained by adding 7 g of distilled water to the sponge-form immunopotentiating composition prepared in Example 3, allowing the mixture to stand overnight followed by kneading.

### Example 6

A gel-form immunopotentiating composition was obtained by adding 7 g of distilled water to the sponge-form immunopotentiating composition prepared in Example 4, allowing the mixture to stand overnight followed by kneading.

### Example 7

A rod-form immunopotentiating composition, in the form of a rod, was obtained by extruding the gel-form immunopotentiating composition prepared in Example 5, followed by drying.

### Example 8

A rod-form immunopotentiating composition, in the form of a rod, was obtained by extruding the gel-form immunopotentiating composition prepared in Example 6, followed by drying.

### Example 9

An aqueous 1 mg/ml avidin solution (11.1 g) and 12.2 g of an aqueous 81 mg/ml human serum albumin (HSA) solution are blended together and the mixture is lyophilised. The lyophilisate is milled and sieved to give a powder with a particle size of not more than 20 micrometers. Separately, 0.7 g of Silastic (registered trademark, Dow Corning Co., USA) medical grade ETR elastomer Q7-4750 part A and 0.7 g of part B are blended together. After blending, the mixture is quickly kneaded with 0.6 g of the powder mentioned above. The kneaded mixture is extruded under pressure through a hole having a diameter of 1.9 mm and allowed to stand at room temperature for curing. The rod is cut to give an immunopotentiating composition.

### Example 10

An aqueous 1 mg/ml avidin solution (11.1 g), 61 microlitres of an aqueous 2 mg/ml IL-1β solution and 12.2 g of an aqueous 81 mg/ml human serum albumin (HSA) solution are blended up and the mixture is lyophilised. The lyophilisate is milled and sieved to give a powder with a particle size of not more than 20 micrometers. Separately, 0.7 g of Silastic (registered trademark, Dow Corning Co., USA) medical grade ETR elastomer Q7-4750 part A and 0.7 g of part B are blended together. After blending, the mixture is quickly kneaded with 0.6 g of the powder mentioned above. The kneaded mixture is extruded under pressure through a hole having a diameter of 1.9 mm and allowed to stand at room temperature for curing. The rod is cut to give an immunopotentiating composition.

### Example 11

The cured product of Example 9 is provided with an outer layer (thickness: 0.2 mm) by immersing in a dispersion of 10% Silastic (registered trade mark, Dow Corning Co., USA) medical grade ETR elastomer Q7-4750 (1:1 mixture of part A and part B) in toluene, followed by drying. The rod is cut to give an immunopotentiating composition.

### Example 12

The cured product of Example 10 is provided with an outer layer (thickness: 0.2 mm) by immersing in a dispersion of 10% Silastic (registered trade mark, Dow Corning Co., USA) medical grade ETR elastomer Q7-4750 (1:1 mixture of part A and part B) in toluene, followed by drying. The rod is cut to give an immunopotentiating composition.

### Example 13

An aqueous 1 mg/ml avidin solution (11.1 g) and 12.2 g of an aqueous 81 mg/ml HSA solution are blended together and the mixture is lyophilised. The lyophilisate is milled and sieved to give a powder with a particle size of not more than 20 micrometers. Separately, 1.372 g of Shin-Etsu Silicone (registered trade mark, Shin-Etsu Chemical Co. Ltd., Japan) KE68 (main material) and 28m g of Shin-Etsu Silicone (registered trade mark, Shin-Etsu Chemical Co., Ltd., Japan) Cat-RC (curing agent) are blended together. After blending, the mixture is quickly kneaded with 0.6 g of the powder mentioned above. The kneaded mixture is extruded under pressure through a hole having a diameter of 1.9 mm and allowed to stand at room temperature for curing. The rod is cut to give an immunopotentiating composition.

### Example 14

An aqueous 1 mg/ml avidin solution (11.1 g), 61 ml of an aqueous 2 mg/ml IL-1β solution and 12.2 g of an aqueous 81 mg/ml HSA solution are blended up and the mixture is lyophilised. The lyophilisate is milled and sieved to give a powder with a particle size of not more than 20 micrometers. Separately, 1.372 g of Shin-Etsu Silicone KE68 (main material) and 28m g of Shin-Etsu Silicone Cat-RC (curing agent) are blended together. After blending, the mixture is quickly kneaded with 0.6 g of the powder mentioned above. The kneaded mixture is extruded under pressure through a hole having a diameter of 1.9 mm and allowed to stand at room temperature for curing. The rod is cut to give an immunopotentiating composition.

### Example 15

The cured product of Example 13 is provided with an outer layer (thickness: 0.2 mm) by immersing in a dispersion of 10% Shin-Etsu Silicone (98:2 mixture of KE-68 and Cat-RC) in toluene, followed by drying. The rod is cut to give an immunopotentiating composition.

### Example 16

The cured product of Example 14 is provided with an outer layer (thickness: 0.2 mm) by immersing in a dispersion of 10% Shin-Etsu Silicone (98:2 mixture of KE-68 and Cat-RC) in toluene, followed by drying. The rod is cut to give an immunopotentiating composition.

### Example 17

An aqueous solution (0.578 g) containing 5 mg/ml of avidin, an aqueous solution (13.0 g) containing 100 mg/ml of sodium citrate and an aqueous solution (13.0 g) containing 100 mg/ml of mannitol were admixed and lyophilised. The lyophilised product was ground in a nitrogen atmosphere to provide a powder. Separately, 1.05 g of Silastic (registered trademark, Dow Corning Co., USA) medical grade ETR elastomer Q7-4750 part A was mixed with 1.05 g of the part B. After blending, the mixture was quickly kneaded with 0.90 g of the above powder. The kneaded mixture was filled into a syringe and extruded under pressure through the 1.6 mm bore and allowed to stand at 25°C for 3 days for curing. The rod was cut to give an immunopotentiating composition.

### Example 18

An aqueous solution (2.89 g) containing 5 mg/ml of avidin, an aqueous solution (6.42 g) containing 100 mg/ml of sodium citrate and an aqueous solution (6.42 g) containing 100 mg/ml of mannitol were admixed and lyophilised. The lyophilised product was ground in a nitrogen atmosphere to provide a powder. Separately, 0.93 g of Silastic (registered trademark, Dow Corning Co., USA) medical grade ETR elastomer Q7-4750 part A was admixed with 0.93 g of part B. After blending, the mixture was quickly kneaded with 0.80 g of the above powder. The kneaded mixture was filled into a syringe and extruded under pressure through the 1.6 mm bore and allowed to stand at 25°C for 3 days for curing. The rod was cut to give an immunopotentiating composition.

### Example 19

A kneaded mixture of avidin, sodium citrate, mannitol and Silastic was filled into a syringe in the same manner as in Example 18. Separately, 50 g of Silastic (registered trademark, Dow Corning Co., USA) medical grade ETR elastomer Q7-4750 part A and 50 g of part B were admixed and filled into another syringe. The fillings were extruded under pressure through concentrically arranged nozzles (outermost diameter: 1.9 mm) so that the drug-containing Silastic formed the inner part and the drug-free Silastic the outer part. The molding was allowed to stand at 37°C for 5 days for curing and then cut to give an immunopotentiating composition.

### Example 20

An aqueous solution (0.30 g) containing 5 mg/ml of avidin, an aqueous solution (4.34 g) containing 100 mg/ml of sodium citrate and an aqueous solution (8.67 g) containing 100 mg/ml of mannitol were admixed and lyophilised. The lyophilised product was ground under a nitrogen atmosphere to provide a powder. Separately, 0.93 g of Silastic (registered trademark, Dow Corning Co., USA) medical grade ETR elastomer Q7-4750 part A was mixed with 0.93 g of the part B. After blending, the mixture was quickly kneaded with 0.80 g of the above powder. The kneaded mixture was filled into a syringe and extruded under pressure through the 1.6 mm bore and allowed to stand at 25°C for 3 days for curing. The molding was cut to give an immunopotentiating composition.

### Example 21

A kneaded mixture of avidin, sodium citrate, mannitol and Silastic was filled into a syringe in the same manner as in Example 20. Separately, 50 g of Silastic (registered trademark, Dow Corning Co., USA) medical grade ETR elastomer Q7-4750 part A and 50 g of part B were admixed and filled into another syringe. The fillings were extruded under pressure through concentrically arranged nozzles (inside diameter of an outer part: 1.9 mm, inside diameter of an inner part: 1.6 mm) so that the drug-containing Silastic formed the inner part and the drug-free Silastic the outer part. The molding was allowed to stand at 25°C for 5 days for curing and then cut to give an immunopotentiating composition.

### Example 22

An aqueous solution (0.45 g) containing 5 mg/ml of avidin, an aqueous solution (3.15 g) containing 2 mg/ml of IL-1 β, an aqueous solution (1.92 g) containing 250 mg/ml of sodium citrate and an aqueous solution (6.19 g) containing 150 mg/ml of mannitol were admixed and lyophilised. The lyophilised product was ground in a nitrogen atmosphere to provide a powder. Separately, 1.05 g of Silastic (registered trademark of Dow Corning Co., USA) medical grade ETR elastomer Q7-4750 part A was mixed with 1.05 g of the part B. After blending, the mixture was quickly kneaded with 0.90 g of the above powder. The kneaded mixture was filled into a syringe and extruded under pressure through the 1.6 mm bore and allowed to stand at 25°C for 5 days for curing. The molding was cut to give an immunopotentiating composition.

### Example 23

A kneaded mixture of avidin, IL-1 β, sodium citrate, mannitol and Silastic was filled into a syringe in the same manner as in Example 22. Separately, 50 g of Silastic (registered trademark of Dow Corning Co., USA) medical grade ETR elastomer Q7-4750 part A and 50 g of part B were admixed and filled into another syringe. The fillings were extruded under pressure through concentrically arranged nozzles (inside diameter of an outer part: 1.9 mm, inside diameter of an inner part: 1.6 mm) so that the drug-containing Silastic formed the inner part and the drug-free Silastic the outer part. The molding was allowed to stand at 25°C for 3 days for curing and then cut to give an immunopotentiating composition.

### (2) Release tests

### Test Example 1

The immunopotentiating composition prepared in Example 7 (10 mg) was placed in 5 ml of phosphate buffer (pH 7.4) containing 0.5% bovine serum albumin and 0.01% sodium azide and the avidin released was assayed by enzyme linked immunosorbant assay (ELISA), and the cumulative release was determined. The results thus obtained are shown in Fig. 1. The immunopotentiating composition released avidin sustainedly over not less than 7 days.

### Test Example 2

The immunopotentiating composition prepared in Example 8 (10 mg) was placed in 5 ml of phosphate buffer (pH 7.4) containing 0.5% bovine serum albumin and 0.01% sodium azide and the avidin and IL-1β released were assayed by ELISA, and the cumulative releases were determined. The results obtained are shown in Fig. 1. The immunopotentiating composition released avidin and IL-1β sustainedly over not less than 7 days.

### (3) Antibody production experiment

### Test Example 3

Two groups of 5 female Balb/C mice received a sub-cutaneous administration of either the immunopotentiating composition prepared in Example 7 containing 100 micrograms of avidin, the immunopotentiating composition prepared in Example 8 containing 100 micrograms of avidin and 20 micrograms of IL-1β. Blood samples were collected from mice on days 7, 14, 21, 35 and 83 post administration. Equal amounts of serum from the five mice in each group were pooled and assayed for anti-avidin specific antibody by ELISA. Results are expressed as 50% mid-point titres and demonstrate the immunopotentiating effects of the compositions (Fig. 2). At 35 days after administration, the antibody titre in blood of the mice given the immunopotentiating composition prepared in Example 7 was about 25 times higher as compared with the antibody titre obtained in the Control Example 1. Furthermore, the antibody titre in blood of the mice given the immunopotentiating composition prepared in Example 8 was as high as about 450 times the antibody titre obtained in the Control Example 1.

### Control Example 1

Five female Balb/C mice received a sub-cutaneous administration of 100 micrograms of soluble avidin in PBS. Blood samples were collected from mice on days 7, 14, 21, 35 and 83 post administration. Equal amounts of serum from the five mice were pooled and assayed for anti-avidin specific antibody by ELISA. Results are expressed as 50% mid-point titres and demonstrate the immunopotentiating effects of the compositions (Fig. 2).

### Test Example 4

Five merino sheep of mixed sex received a sub-cutaneous administration of the immunopotentiating composition produced in Example 8. Blood samples were collected from sheep on days 7, 14, 21, and 35 post administration. Equal amounts of serum from the five sheep were pooled and assayed for anti-avidin specific antibody by ELISA. Results are expressed as 50% mid-point titres and demonstrate the immunopotentiating effects of the compositions (Fig. 3). A high level of anti-avidin specific antibody production was established at 14 days after administration.

### Control Example 2

Five merino sheep of mixed sex received a sub-cutaneous administration of 100 micrograms of soluble avidin in PBS. Blood samples were collected from sheep on days 7, 14, 21, and 35 post administration. Equal amounts of serum from the five sheep were pooled and assayed for anti-avidin specific antibody by ELISA. Results are expressed as 50% mid-point titres and demonstrate the immunopotentiating effects of the compositions (Fig. 3). Anti-avidin specific antibody production was not observed for 35 days after administration.

### Control Example 3

Five merino sheep of mixed sex received a sub-cutaneous administration of 100 micrograms of soluble avidin and 20 micrograms of IL-1β in PBS. Blood samples were collected from sheep on days 7, 14, 21, and 35 post administration. Equal amounts of serum from the five sheep were pooled and assayed for anti-avidin specific antibody by ELISA. Results are expressed as 50% mid-point titres and demonstrate the immunopotentiating effects of the compositions (Fig. 3). Anti-avidin specific antibody production was not observed for 35 days after administration.

### (4) Histological analysis

Sheep received a sub-cutaneous administration of either the immunopotentiating composition prepared in Example 7 or the immunopotentiating composition prepared in Example 8, into three distinct sites on the left flank. Animals were sacrificed and skin biopsies recovered for analysis at 72 hours after the administration. Biopsies were embedded in OCT for analysis of cell surface CD45 expression on frozen sections. The histological micrograph demonstrate the infiltration of leukocytes induced by the immunopotentiating compositions (Fig. 4 and 5).

### Test Example 5

The immunopotentiating composition prepared in Example 17 and cut to a size corresponding to an avidin content of 10 micrograms and the immunopotentiating compositions prepared in Examples 18 and 19 and each cut to a size corresponding to an avidin content of 100 micrograms were respectively placed in 2 ml of phosphate buffer (pH 7.4) containing 0.3% of Tween 20 and 0.01% of sodium azide and allowed to stand. The avidin released was assayed by ELISA and the cumulative release was determined. The results obtained are shown in Fig. 6. The release kinetics of avidin could be controlled by selecting the form of the composition. Thus, the matrix-form compositions (Examples 17 and 18) showed an approximately first-order release pattern while the covered-rod form composition (Example 19) showed an approximately zero-order release pattern. These compositions released avidin sustainedly over at least 30 days.

### Test Example 6

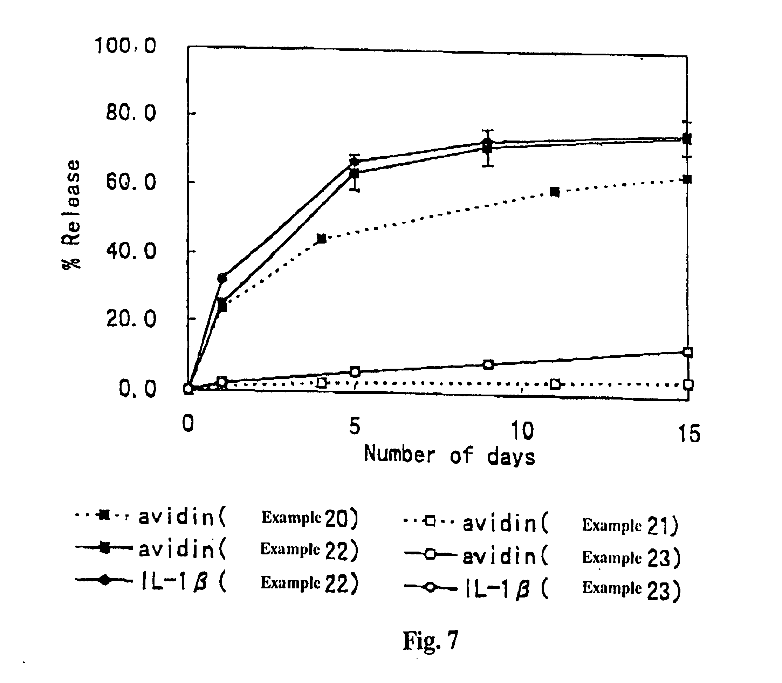

The immunopotentiating compositions prepared in Examples 20 and 21 and each cut to a size corresponding to an avidin content of 5 micrograms and the immunopotentiating compositions prepared in Examples 22 and 23 and each cut to a size corresponding to an avidin content of 5 micrograms and an IL-1β content of 5 micrograms were respectively placed in 2 ml of phosphate buffer (pH 7.4) containing 0.3% of Tween 20 and 0.01% of sodium azide and allowed to stand. The avidin and IL-1β released were assayed by ELISA and the cumulative releases were determined. The results obtained are shown in Fig. 7. As in Test Example 5, the release kinetics of avidin and IL-1β could be controlled by selecting the form of the composition. These compositions released avidin and IL-1β sustainedly over at least 15 days.

### Test Example 7

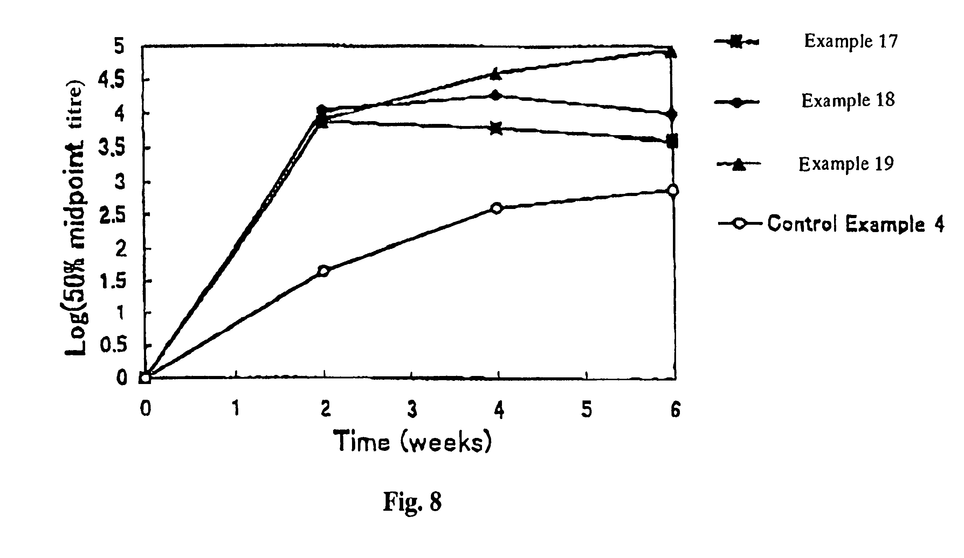

Three groups of Balb/C mice (six males per group) received subcutaneous administration of the immunopotentiating composition prepared in Example 17 (containing 10 micrograms of avidin), the composition prepared in Example 18 (containing 100 micrograms of avidin), and the composition prepared in Example 19 (containing 100 micrograms of avidin), respectively. Blood samples were collected at 14, 28 and 42 days after administration. At each time of collection, aliquots of sera from the six mice in each group were pooled and assayed for anti-avidin antibody titre by ELISA. Each antibody titre was expressed in the 50% midpoint titre. The results thus obtained are shown in Fig. 8. At 14 days after administration, the blood antibody titre of the mice given the immunopotentiating composition prepared in Example 17 reached a level about 180 times as high as that in Control Example 4 in spite of the fact that the quantity of avidin was only one-tenth of the quantity in the composition of Control Example 4. At 14 days after administration, the antibody titres in blood of the mice given the immunopotentiating compositions prepared in Examples 18 and 19 were about 250 and about 190 times higher as compared with that of Control Example 4. The blood antibody titres in the mice given the immunopotentiating compositions prepared in Examples 17, 18 and 19 were higher than that of Control Example 4 over 6 weeks following administration.

### Control Example 4

Balb/C mice (6 males) were subcutaneously given a PBS solution containing 100 micrograms of avidin. At 14, 28 and 42 days after administration, blood samples were collected. At each time of collection, aliquots of sera from the six mice were pooled and assayed for anti-avidin antibody titre by ELISA. Each antibody titre was expressed in the 50% midpoint titre. The results obtained are shown in Fig. 8.

### Test Example 8

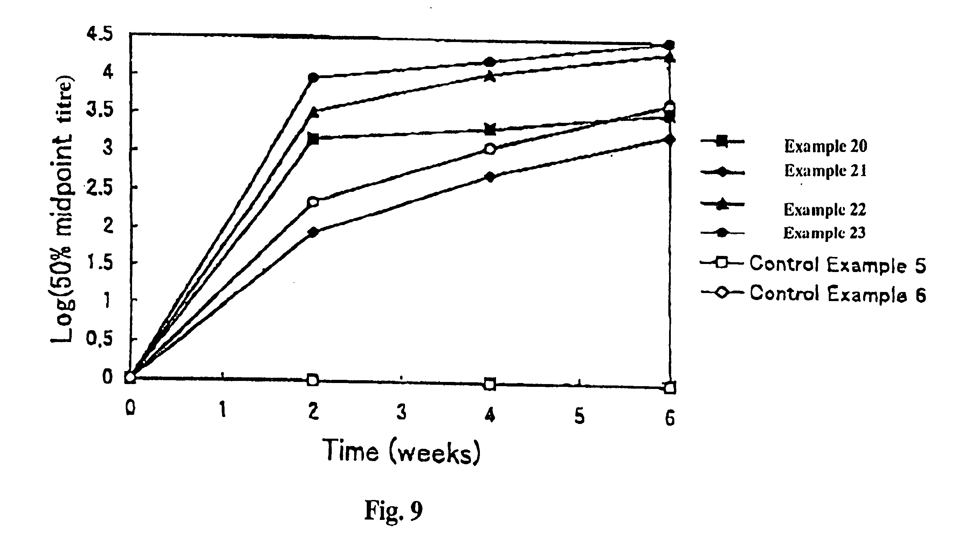

Four groups of Balb/C mice (six males per group) received subcutaneous administration of the immunopotentiating composition prepared in Example 20 (containing 5 micrograms of avidin), the composition prepared in Example 21 (containing 5 micrograms of avidin), the composition prepared in Example 22 (containing 5 micrograms of avidin and 5 micrograms of IL-1β), and the composition prepared in Example 23 (containing 5 micrograms of avidin and 5 micrograms of IL-1 β), respectively. Blood samples were collected at 14, 28 and 42 days after administration. At each time of collection, aliquots of sera from the six mice in each group were pooled and assayed for anti-avidin antibody titre by ELISA. Each antibody titre was expressed in the 50% midpoint titre. The results thus obtained are shown in Fig. 9. In the mice given the immunopotentiating compositions prepared in Examples 20, 21, 22 and 23, anti-avidin antibody was detected in blood from 14 days after administration whereas the blood antibody titre in mice given the same amount of avidin in Control Example 5 was below the detection limit throughout the test period. The blood antibody titres in the mice given the immunopotentiating compositions of Examples 20, 22 and 23 were higher than that of Control Example 6 until 28 days after administration, and the blood antibody titres in the mice given the immunopotentiating compositions of Examples 22 and 23 were much higher than that of Control Example 5 throughout the test period.

### Control Example 5

Balb/C mice (6 males) were subcutaneously given a PBS solution containing 5 micrograms of avidin. At 14, 28 and 42 days after administration, blood samples were collected. At each time of collection, aliquots of sera from the six mice were pooled and assayed for anti-avidin antibody titre by ELISA. Each antibody titre was expressed in the 50% midpoint titre. The results obtained are shown in Fig. 9.

### Control Example 6

Balb/C mice (6 males) were subcutaneously given a PBS solution containing 5 micrograms of avidin and 0.26% (by weight) of alum. At 14, 28 and 42 days after administration, blood samples were collected. At each time of collection, aliquots of sera from the six mice were pooled and assayed for anti-avidin antibody titre by ELISA. Each antibody titre was expressed in the 50% midpoint titre. The results are shown in Fig. 9.

Hereafter, the "immunopotentiating composition" is abbreviated as IC.

### Test Example 9

A silicone-based IC was prepared in a manner similar to that described in Examples 20 and 22 above. The silicon-based IC is identified as "matrix" in the tables below. The contents of each composition are shown in Table 1 below.

Similarly a coated silicon-based IC was prepared in a manner similar to that described in Examples 21 and 23 above. The coated silicon-based IC is identified as "covered rod" in the tables below. The contents of each composition are shown in Table 1 below.

**Table 1**

| **Contents of the compositions for evaluation of immunoenhancing effect of silicone based ICs in test Example 9** | | | |
|---|---|---|---|
| **Group** | | **Composition (µg)** | |
| | | **IL-1 β** | **Avidin** |
| 1 | matrix | 0 | 500 |
| 2 | matrix | 0 | 100 |
| 3 | matrix | 0 | 10 |
| 4 | matrix | 20 | 500 |
| 5 | matrix | 20 | 100 |
| 6 | matrix | 20 | 10 |
| 7a | covered rod | 50 | 500 |
| 7b | matrix | 50 | 500 |
| 8 | matrix | 50 | 100 |
| 9 | matrix | 50 | 10 |
| 10 | 500 µg avidin in PBS (Phosphate Buffered Solution) | | |
| 11 | 500 µg avidin + 20 µg IL-1 β in PBS | | |
| 12 | 500 µg avidin in alum | | |

In this trial, the silicone based immunopotentiating compositions or control compositions (groups 10, 11, 12) were introduced by the subcutaneous route followed by a secondary immunisation of 100 µg of avidin in PBS at day 28.

### Efficacy of Silicone Based Immunopotentiating composition

Sheep were divided into 12 groups with 7 sheep/group.

Each received a subcutaneous administration of a composition according to Table 1. A secondary immunisation with 100 µg of avidin occurred at day 28. The results obtained are shown in Table 2.

**Table 2**

| **Anti-avidin antibody titres in sheep as found in Test Example 9 (50% midpoint titres)** | | | | | | | |
|---|---|---|---|---|---|---|---|
| | **Gp.** | **Composition (µg)** | | **Post-Primary** | | **Post-Secondary** | |
| | | **IL-1β** | **Avidin** | **14d** | **28d** | **14d** | **28d** |
| M | 1 | 0 | 500 | **500** | **160** | **500** | **398** |
| M | 2 | 0 | 100 | **795** | **500** | **398** | **50** |
| M | 3 | 0 | 10 | **560** | **630** | **1260** | **1260** |
| M | 4 | 20 | 500 | **1585** | **1000** | **2510** | **1260** |
| M | 5 | 20 | 100 | **2000** | **1000** | **2000** | **1260** |
| M | 6 | 20 | 10 | **2000** | **1780** | **4467** | **3980** |
| M | 7b | 50 | 500 | **2820** | **1585** | **6310** | **4467** |
| M | 8 | 50 | 100 | **560** | **560** | **5012** | **2512** |
| M | 9 | 50 | 10 | **6310** | **6310** | **10000** | **6310** |
| CR | 7a | 50 | 500 | **7080** | **6310** | **19950** | **19950** |
| S | 9 | 0 | 500 | **200** | **<50** | **<50** | **<50** |
| S | 10 | 20 | 500 | **1000** | **316** | **6310** | **1585** |
| A | 11 | 0 | 500 | **1585** | **1585** | **5012** | **2512** |

| | | | | | | | |
|---|---|---|---|---|---|---|---|
| M: matrix CR: covered rod S: PBS solution A: PBS solution containing alum | | | | | | | |

The anti-avidin specific antibody released was determined by ELISA with titrations conducted from pooled serum from each group.

In the absence of IL-1β adjuvant, the matrix silicone IC was superior to antigen delivered in saline (at all doses tested) but less effective than antigen delivered in alum.

The antibody response was enhanced by the addition of IL-1β as adjuvant in both silicone and saline compositions. Several silicone IC with IL-1β induced responses superior to the avidin in alum composition.

For matrix silicone IC, there was a trend towards the lowest antigen dose being most effective for induction of high antibody responses.

The covered rod IC was superior to the matrix IC, based on antibody titre and duration of response.

### Biocompatibility of Silicone IC

Sheep were divided into 8 groups with 7 sheep per group.

Each received a subcutaneous administration of a silicone IC according to Table 3.

**Table 3**

| **Contents of the silicone based ICs for evaluation of biocompatibility in Test Example 9** | | | |
|---|---|---|---|
| **Group** | | **Composition (µg)** | |
| | | **IL-1** | **Avidin** |
| 1 | Matrix | 0 | 0 |
| 2 | Matrix | 0 | 500 |
| 3 | Matrix | 50 | 0 |
| 4 | Matrix | 50 | 500 |
| 5 | Covered rod | 0 | 500 |
| 6 | Covered rod | 50 | 0 |
| 7 | Covered rod | 50 | 500 |

Two sheep per group monitored for white cell counts and body temperature.

Biopsies of implant sites collected for histology at:
2 days post implant (2 sheep)
4 weeks post implant (2 sheep)
8 weeks post implant (2 sheep)

At two days after implantation, mild oedema was observed at the site of all IC. This was more obvious in IC with IL-1β.

At 4 and 8 week post implantation, tissues surrounding the IC sites appeared normal. The IC were not encapsulated and were not adhering to the tissues, but could be moved freely. No adverse tissue reactions were observed.

### Systemic Effects

Body temperature and white blood cell counts were undertaken.

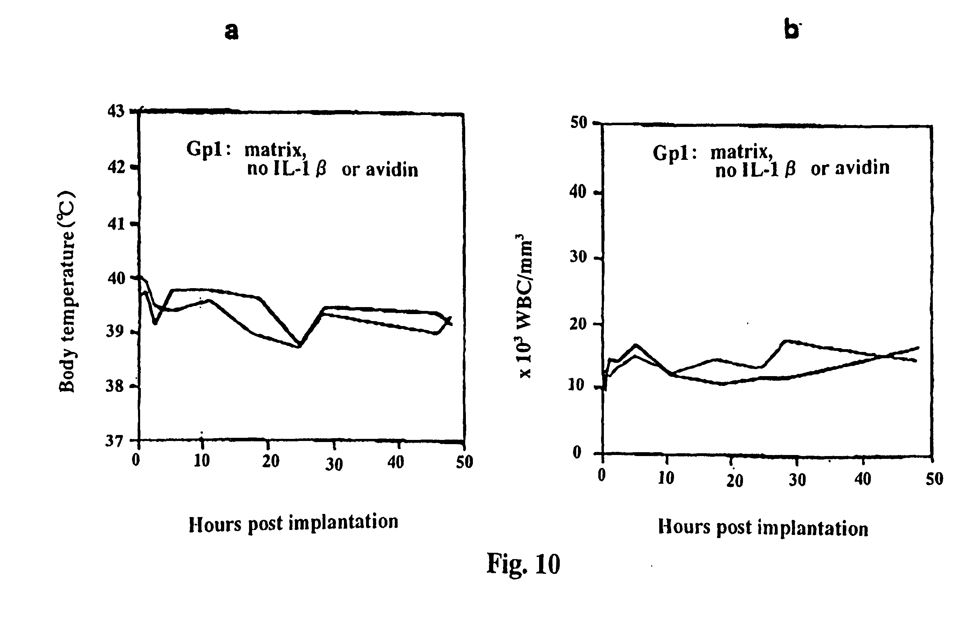

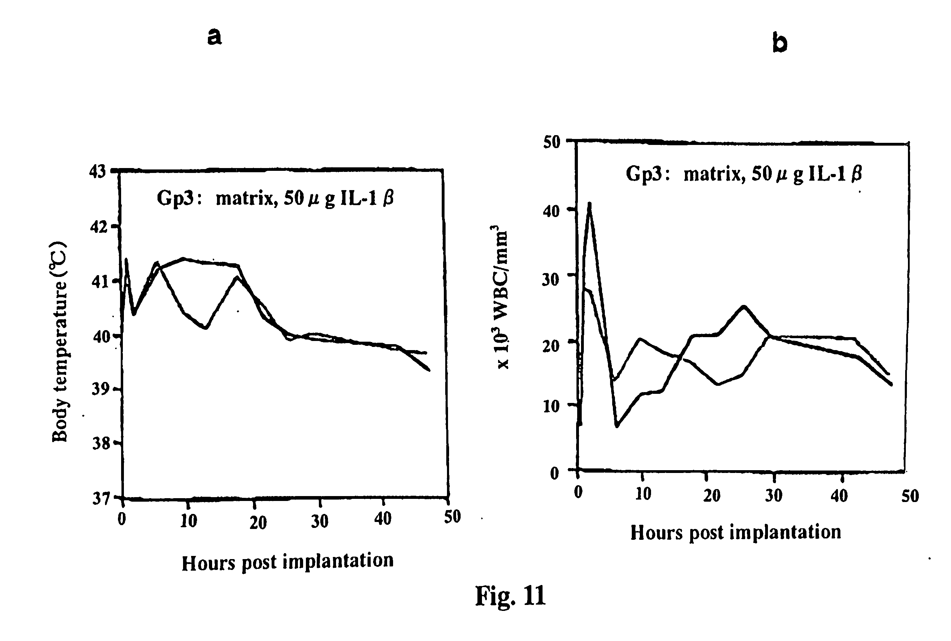

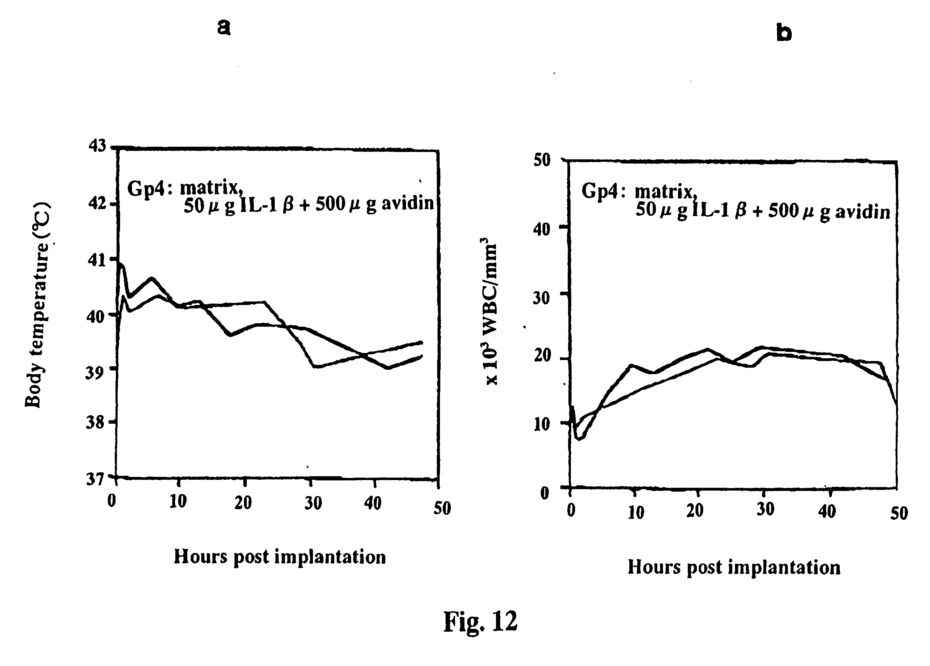

The results obtained are shown for silicone IC alone in Figures 10a and b, for IC incorporating IL-1β only in Figures 11a and b and for IC with avidin and IL-1β in Figures 12a and b.

All types of silicone implants which did not incorporate IL-1β did not induce any adverse effects on body temperature or white cell count.

When silicone IC incorporating IL-1β only was administrated, transient increases in body temperature were observed in sheep which were less severe than results observed following injection of IL-1β in saline. The mean increase in these sheep was 1°C, and normal temperatures were observed by 24 hours. Furthermore, white cell counts increased up to 4-fold normal levels, however normal levels were again observed after 24 hours.

The presence of avidin in the silicone IC resulted in a reduction in both the severity and persistence of white blood cell counts (WBC) and temperature changes associated with IL-1β release from the IC.

These results demonstrate that the silicone ICs do not induce long term aberrations in body temperature and white cell counts, and the transient fluctuations which were observed were minimal, and related to the inclusion of IL-1β in the ICs rather than any activity of the silicone ICs themselves.

### Immunohistological assessment of administration sites.

Results for the different types of silicone ICs were identical; the differing responses were attributable only to the presence or absence of IL-1β in the ICs.

### 1. ICs incorporating IL-1β.

DAY 2 In all animals which received ICs containing IL-1β there was a massive influx of cells, mostly neutrophils, around the administration site and throughout the surrounding tissue.

Cells staining for T and B cell markers were mainly in the epidermis and only a few scattered in the lower layers of the skin.
WEEK 4 Immune cells observed in these sections were mainly neutrophils. An increase in MHC Class I and II positive cells over the level observed at day two was evident. A few CD4, CD8, γ δ and CD1 positive cells occurred in the layer around the IC and scattered through the rest of the tissue. Some CD45R positive cell are also appearing scattered through the skin.
WEEK 8 Cells were now mainly as a layer around the IC, and the tissue had a more normal appearance.

Cells positive for LCA surrounded the ICs and a cell layer which did not stain with any of the lymphocyte markers was also evident. It is likely that these cells were fibroblasts. Paraffin sections stained with Masson's trichrome showed a thin layer of densely staining collagen, indicating the onset of encapsulation of the IC. MHC Class I and II positive cells also occurred in the LCA-positive layer around the IC but not throughout the tissue as in week four. There are only scattered positives for CD4, less CD8, γ δ, CD1 and CD45R.

### 2. ICs without IL-1β.

DAY 2 Biopsies taken from animals which received ICs that did not include IL-1β all had the appearance of normal skin. No cellular influx or oedema was apparent. Minimal lymphocyte surface marker staining occurred in cells in the epidermis.
WEEK 4 Fibroblasts were evident around the administration sites, and in addition, the covered rod type IC showed LCA positive cells at the open end of the IC. These cells were mainly MHC Class I positive with a few Class II and CD4 positives.
WEEk 8 There were some fibroblasts around the IC, and cells staining with any of the lymphocyte surface markers were rare.

These results indicate that the silicone ICs were well tolerated following subcutaneous administration into sheep and no adverse reactions which would limit their usage was observed after 8 weeks of administration.

### Test Example 10

Test Example 9 was repeated utilizing a collagen-based IC, which was prepared in a manner similar to that described in Examples 7 and 8. The composition of the IC are shown in Table 4 below and the results obtained are shown in Table 5 below.

**Table 4**

| **Contents of the compositions for determination of dependence of immunoenhancing effect of collagen based ICs on amounts of antigen and cytokine in Test Example 10** | | |
|---|---|---|
| **Group** | **Composition (µg)** | |
| | **IL-1β** | **Avidin** |
| 1 | 0 | 500 |
| 2 | 0 | 100 |
| 3 | 0 | 10 |
| 4 | 2 | 500 |
| 5 | 2 | 100 |
| 6 | 2 | 10 |
| 7 | 20 | 500 |
| 8 | 20 | 100 |
| 9 | 20 | 10 |
| 10 | 50 | 500 |
| 11 | 50 | 100 |
| 12 | 50 | 10 |

| | | |
|---|---|---|
| * subcutaneous route * secondary immunisation of 100 µg of avidin in PBS | | |

**Table 5**

| **Dependence of immunoenhancing effect of collagen based IC on amounts of antigen and cytokine in Test Example 10 (50% midpoint titres)** | | | | | | |
|---|---|---|---|---|---|---|
| **Gp.** | **Composition (µg)** | | **Post-Primary** | | **Post-Secondary** | |
| | **IL-1** | **Avidin** | **14d** | **28d** | **14d** | **28d** |
| 1 | 0 | 500 | **500** | **315** | **315** | **315** |
| 2 | 0 | 100 | **80** | **100** | **315** | **160** |
| 3 | 0 | 10 | **1000** | **630** | **630** | **630** |
| 4 | 2 | 500 | **2000** | **700** | **630** | **400** |
| 5 | 2 | 100 | **2000** | **795** | **630** | **250** |
| 6 | 2 | 10 | **1000** | **795** | **795** | **500** |
| 7 | 20 | 500 | **1580** | **795** | **795** | **795** |
| 8 | 20 | 100 | **2000** | **1000** | **1260** | **1000** |
| 9 | 20 | 10 | **1000** | **700** | **500** | **400** |
| 10 | 50 | 500 | **2000** | **1260** | **2000** | **1260** |
| 11 | 50 | 100 | **2500** | **2000** | **2000** | **1580** |
| 12 | 50 | 100 | **400** | **200** | **630** | **630** |

The results confirm that the inclusion of IL-1β into the collagen IC enhances the antibody response to avidin.

The optimum dose of IL-1β could not be statistically established, however the highest response were recorded when the two highest doses of IL-1β were administered.

Results achieved with the collagen IC were compared with alum and PBS (see Table 6).

**Table 6**

| **Contents of the compositions for evaluation of immunoenhancing effect of collagen based IC in Test Example 10** | | | |
|---|---|---|---|
| **Group** | | **Composition (µg)** | |
| | | **IL-1β** | **Avidin** |
| 1 | IC | 0 | 500 |
| 2 | IC | 20 | 500 |
| 3 | In alum | 0 | 500 |
| 4 | In alum | 20 | 500 |
| 5 | In PBS | 0 | 500 |
| 6 | IC | 20 | 500 i.m. |
| 7 | In alum | 20 | 500 i.m. |

| | | | |
|---|---|---|---|
| i.m.: intramuscular administration | | | |

Subcutaneous route was used, except where indicated and secondary administration was undertaken with 100 µg of avidin in PBS. Twenty-eight days after the secondary immunization, delayed type hypersensitivity responses were examined by injecting 1 µg of avidin in PBS intradermally to the wool-free region of the inner thigh of sheep. The site was examined at 24 and 48 hours after injection for oedema and erythema.

The results obtained are shown in Tables 7 and 8.

**Table 7**

| **Anti-avidin antibody titres in sheep as found in Test Example 10 (50% midpoint titres)** | | | | | | | | | |
|---|---|---|---|---|---|---|---|---|---|
| **Gp.** | **Composition (µg)** | | **Post-Primary** | | **Post-Secondary** | | | | |
| | **IL-1β** | **Avidin** | **14d** | **28d** | **14d** | **28d** | **43d** | **53d** | **76d** |
| 1 | 0 | 500 | 200 | 126 | 126 | 100 | 100 | 100 | 80 |
| | IC | | | | | | | | |
| 2 | 20 | 500 | 400 | 282 | 200 | 891 | 1631 | 251 | 80 |
| | IC | | | | | | | | |
| 3 | 0 | 500 | 1000 | 1000 | 3163 | 251 | 251 | 158 | 126 |
| | In alum | | | | | | | | |
| 4 | 20 | 500 | 5012 | 5012 | 8913 | 1778 | 1259 | 1000 | 80 |
| | In alum | | | | | | | | |
| 5 | 0 | 500 | 159 | 50 | 159 | 159 | 159 | 159 | 794 |
| | In PBS | | | | | | | | |
| 6 | 20 | 500 | 2512 | 1000 | 631 | 251 | 251 | 158 | 126 |
| | IC, i.m. | | | | | | | | |
| 7 | 20 | 500 | 5012 | 1995 | 1995 | 1259 | 794 | 794 | 126 |
| | In alum, i.m. | | | | | | | | |

**Table 8**

| **Score of Delayed-type hypersensitivity in Test Example 10** | | | | | | |
|---|---|---|---|---|---|---|
| **Gp** | **Composition** | **#** | **24 hour** | | **48 hour** | |
| | | | **E** | **O** | **E** | **O** |
| 1 | 500 µg avidin collagen IC | G1 | - | - | - | - |
| | | G2 | - | - | - | - |
| | | G3 | - | - | + | - |
| | | G4 | - | - | - | - |
| | | G5 | + | - | - | - |
| | | G6 | - | - | + | - |
| | | G7 | + | - | - | - |
| 2 | 500 µg avidin +20 µg IL-1β collagen IC | G8 | - | - | - | - |
| | | G9 | - | - | - | - |
| | | G10 | - | - | - | - |
| | | G11 | - | - | - | - |
| | | G12 | - | - | - | - |
| | | G13 | - | - | - | - |
| | | G14 | - | - | - | + |
| 3 | 500 µg avidin in alum | G15 | - | - | - | - |
| | | G16 | + | - | - | - |
| | | G17 | + | - | + | - |
| | | G18 | ++ | + | ++ | + |
| | | G19 | + | - | + | - |
| | | G20 | + | - | + | + |
| | | G21 | +++ | + | ++ | + |
| 4 | 500 µg avidin + 20 µg IL-1β in alum | G22 | - | - | - | - |
| | | G23 | - | - | + | + |
| | | G24 | + | - | + | - |
| | | G25 | + | ++ | - | - |
| | | G26 | + | - | + | - |
| | | G27 | + | - | + | - |
| | | G28 | + | - | - | - |
| 5 | 500 µg avidin in PBS | G29 | +++ | + | ++ | - |
| | | G30 | - | - | - | - |
| | | G31 | + | - | - | - |
| | | G32 | - | - | - | - |
| | | G33 | + | - | + | - |
| | | G34 | ++ | + | - | - |
| | | G35 | - | - | - | - |
| 6 | I.M 500 µg avidin + 20 µg IL-1β collagen IC | G36 | + | - | + | - |
| | | G37 | - | - | - | - |
| | | G38 | + | - | - | - |
| | | G39 | - | - | - | - |
| | | G40 | - | - | - | - |
| | | G41 | + | + | + | - |
| | | G42 | - | - | - | - |
| 7 | I.M. | G43 | - | - | - | - |
| | 500 µg avidin + 20 µg IL-1β in alum | G44 | - | - | - | - |
| | | G45 | - | - | - | - |
| | | G46 | + | - | + | - |
| | | G47 | ++ | + | + | - |
| | | G48 | ++ | + | | - |
| | | G49 | + | - | | - |

| | | | | | | |
|---|---|---|---|---|---|---|
| E: erythema ○: oedema | | | | | | |

The collagen IC did not elicit any strong delayed type hypersensitivity reactions.

Mild DTH reactions were recorded for sheep immunised with liquid compositions.

No immediate hypersensitivity reactions were observed in any animal.

### Test Example 11

Test Examples 9 and 10 were repeated to assess single shot immunisation effects, utilising IC as specified in Table 9. The results obtained are shown in Tables 10 and 11.

**Table 9**

| **Contents of the compositions for evaluation of immunoenhancing effect with single shot immunization in Test Example 11** | | | |
|---|---|---|---|
| **Group** | | **Composition (µg)** | |
| | | **IL-1β** | **Avidin** |
| 1 | collagen IC | 0 | 500 |
| 2 | collagen IC | 50 | 100 |
| 3 | collagen IC | 50 | 500 |
| 4 | silicone matrix IC | 0 | 500 |
| 5 | silicone matrix IC | 50 | 100 |
| 6 | silicone matrix IC | 50 | 500 |
| 7 | in alum | 0 | 500 |
| 8 | in alum | 50 | 100 |
| 9 | in alum | 50 | 500 |
| 10 | in PBS | 0 | 500 |
| 11 | silicone covered rod IC | 0 | 500 |
| 12 | silicone covered rod IC | 50 | 500 |

| | | | |
|---|---|---|---|
| * subcutaneous route | | | |

**Table 10**

| **Anti-avidin antibody titres in sheep as found in Test Example 11 (50% midpoint titres)** | | | | | | | | | | |
|---|---|---|---|---|---|---|---|---|---|---|
| | **Gp.** | **Composition (µg)** | | **Post-Primary** | | | | | **Post-Secondary** | |
| | | **IL-1** | **Avidin** | **14d** | **28d** | **42d** | **56d** | **69d** | **10d** | **33d** |
| Cl | 1 | 0 | 500 | **158** | **126** | **<50** | **<50** | **<50** | **<50** | **<50** |
| Cl | 2 | 50 | 100 | **224** | **158** | **200** | **100** | **100** | **281** | **158** |
| Cl | 3 | 50 | 500 | **631** | **282** | **126** | **50** | **50** | **158** | **158** |
| M | 4 | 0 | 500 | **158** | **100** | **<50** | **<50** | **<50** | **<50** | **<50** |
| M | 5 | 50 | 100 | **1000** | **562** | **200** | **<50** | **<50** | **199** | **158** |
| M | 6 | 50 | 500 | **631** | **316** | **178** | **80** | **126** | **794** | **398** |
| A | 7 | 0 | 500 | **891** | **501** | **251** | **158** | **158** | **794** | **630** |
| A | 8 | 50 | 100 | **1585** | **1259** | **562** | **251** | **251** | **1000** | **630** |
| A | 9 | 50 | 500 | **7943** | **2239** | **1000** | **398** | **398** | **1000** | **398** |
| S | 10 | 0 | 500 | **158** | **80** | **<50** | **<50** | **<50** | **158** | **<50** |
| CR | 11 | 0 | 500 | **251** | **126** | **<50** | **<50** | **<50** | **<50** | **<50** |
| CR | 12 | 50 | 500 | **7943** | **3162** | **1778** | **1259** | **1259** | **3162** | **1259** |

| | | | | | | | | | | |
|---|---|---|---|---|---|---|---|---|---|---|
| Cl: collagen IC M: matrix IC A: PBS solution containing alum S: PBS solution CR: covered rod IC | | | | | | | | | | |

**Table 11**

| **Score of Delayed-type hypersensitivity in Test Example 11** | | | | | | |
|---|---|---|---|---|---|---|
| **Gp** | **Composition** | **#** | **24 hour** | | **48 hour** | |
| | | | **E** | **O** | **E** | **O** |
| 1 | 500 µg avidin collagen IC | Y1 | - | - | - | - |
| | | Y2 | - | - | - | - |
| | | Y3 | - | - | - | - |
| | | Y4 | - | - | - | - |
| | | Y5 | + | - | + | - |
| | | Y6 | - | - | - | - |
| | | Y7 | - | - | - | - |
| 2 | 100 µg avidin +50 µg IL-1β collagen IC | Y8 | - | - | - | - |
| | | Y9 | - | - | - | - |
| | | Y10 | - | - | - | - |
| | | Y11 | - | - | - | - |
| | | Y12 | - | - | - | - |
| | | Y13 | - | - | - | - |
| | | Y14 | ++++ | - | +++ | - |
| 3 | 500 µg avidin + 50 µg IL-1β collagen IC | Y15 | - | - | - | - |
| | | Y16 | - | - | - | - |
| | | Y17 | - | - | - | - |
| | | Y18 | ++ | ++ | - | - |
| | | Y19 | - | - | - | - |
| | | Y20 | - | - | - | - |
| | | Y21 | - | - | - | - |
| 4 | 500 µg avidin silicone matrix IC | Y22 | - | - | - | - |
| | | Y23 | - | - | - | - |
| | | Y24 | - | - | - | - |
| | | Y25 | - | - | - | - |
| | | Y26 | - | - | - | - |
| | | Y27 | - | - | - | - |
| | | Y28 | - | - | - | - |
| 5 | 100 µg avidin + 50 µg IL-1β silicone matrix IC | Y29 | - | - | - | - |
| | | Y30 | - | - | - | - |
| | | Y31 | - | - | - | - |
| | | Y32 | - | - | - | - |
| | | Y33 | - | - | - | - |
| | | Y34 | - | - | - | - |
| | | Y35 | - | - | - | - |
| 6 | 500 µg avidin + 50 µg IL-1β silicone matrix IC | Y36 | - | - | - | - |
| | | Y37 | - | - | - | - |
| | | Y38 | - | - | - | - |
| | | Y39 | - | - | - | - |
| | | Y40 | - | - | - | - |
| | | Y41 | - | - | - | - |
| | | Y42 | - | - | - | - |
| 7 | 500 µg avidin in alum | Y43 | - | - | - | - |
| | | Y44 | - | - | - | - |
| | | Y45 | +++ | ++ | - | +++ |
| | | Y46 | + | - | + | - |
| | | Y47 | + | + | ++ | + |
| | | Y48 | + | - | - | - |
| | | Y49 | + | - | + | - |
| 8 | 100 µg avidin + 50 µg IL-1β in alum | Y50 | - | - | - | - |
| | | Y51 | - | - | - | - |
| | | Y52 | - | - | - | - |
| | | Y53 | ++ | ++ | +++ | ++ |
| | | Y54 | - | - | - | - |
| | | Y55 | ++ | + | ++ | - |
| | | Y56 | + | + | + | + |
| 9 | 500 µg avidin + 50 µg IL-1β in alum | Y57 | +++ | - | ++ | + |
| | | Y58 | - | - | - | - |
| | | Y59 | - | - | + | - |
| | | Y60 | + | - | + | - |
| | | Y61 | - | - | - | - |
| | | Y62 | ++ | ' + | ++ | - |
| | | Y63 | + | - | + | - |
| 10 | 500 µg avidin in PBS | Y64 | - | - | - | - |
| | | Y65 | - | - | - | - |
| | | Y66 | - | - | - | - |
| | | Y67 | - | - | - | - |
| | | Y68 | - | - | - | - |
| | | Y69 | - | - | - | - |
| | | Y70 | - | - | - | - |
| 11 | 500 µg avidin silicone covered rod IC | Y71 | + | - | - | - |
| | | G63 | - | - | - | - |
| | | G64 | - | - | - | - |
| | | G65 | - | - | - | - |
| | | G66 | - | - | - | - |
| | | G67 | - | - | - | - |
| 12 | 500 µg avidin + 50 µg IL-1β silicone covered rod IC | G68 | - | - | - | - |
| | | G69 | - | - | - | - |
| | | G70 | - | - | - | - |
| | | G71 | - | - | - | - |
| | | G72 | - | - | - | - |
| | | G73 | + | - | + | - |
| | | G74 | + | - | + | - |

The collagen and silicone IC did not elicit any strong delayed type hypersensitivity reactions.

Mild DTH reactions were recorded for sheep immunized with liquid compositions.

No immediate hypersensitivity reactions were observed in any animal.

The covered rod silicone IC was the most effective formulation for single dose immunization, inducing both the highest titres and the most persistent response.

The effective use of the covered rod IC for immunization was dependent on the inclusion of IL-1β.

The covered rod IC did not inherently induce delayed type hypersensitivity responses.

An effective memory response was induced in animals which received ICs incorporating IL-1 β. This is indicated by the response after the secondary immunization was given at day 69, after the titres for the first immunization had declined.

Isotyping analysis was also performed on these serum samples. Results indicated high levels of IgG were present throughout the course of the experiment in all groups, while IgM was detectable at low levels only at the 14 day time point. This indicates isotype switching has occurred effectively after only a single administration of antigen.

### Test Example 12

Test Example 11 was repeated utilizing a series of antigens which can prevent infection of disease in place of the model avidin antigen, as specified in Table 12.

**Table 12**

| **Contents of the compositions for evaluation of immunoenhancing effect with clostridial antigens in Test Example 12** | |
|---|---|
| **Gp** | **Formulation** |
| 1 | Tetanus toxoid + 50 µg IL-1β in collagen IC |
| 2 | Tetanus toxoid + 50 µg IL-1β in covered rod silicone IC |
| 3 | Tetanus toxoid + 50 µg IL-1β in alum |
| 4 | Tetanus toxoid in alum |
| 5 | novyi toxoid + 50 µg IL-1β in collagen IC |
| 6 | novyi toxoid + 50 µg IL-1β in covered rod silicone IC |
| 7 | novyi toxoid + 50 µg IL-1β in alum |
| 8 | novyi toxoid in alum |

**Table 13**

| **Anti-clostridial antigen antibody titres in sheep as found in Test Example 12 (50% midpoint titres)** | | | | | |
|---|---|---|---|---|---|
| Gp. | Post-Primary | | Post-Secondary | | |
| | 14d | 28d | 14d | 27d | 50d |
| 1 | 562 | 251 | 1000 | 794 | 501 |
| 2 | 1995 | 1000 | 7080 | 2511 | 1580 |
| 3 | 1584 | 891 | 2512 | 1259 | 631 |
| 4 | 891 | 631 | 1259 | 794 | 398 |
| 5 | 891 | 501 | 5623 | 1412 | 891 |
| 6 | 2512 | 1585 | 15849 | 5012 | 3163 |
| 7 | 891 | 282 | 2512 | 1259 | 891 |
| 8 | 1259 | 501 | 1412 | 631 | 355 |

The results obtained are shown in Table 13.

The covered rod IC is clearly superior to both the conventional alum vaccine and the alum/IL-1β combination for both antigens tested. Collagen IC induced titres that were not significantly different from the alum formulation, however at later time points, the covered rod IC with C, novyi toxoid induced titres which more than 2-fold higher than for the alum formulation.

### Test Example 13

A dose response analysis was conducted utilising silicone-based IC. The composition and structure of each IC are shown in Table 14 below. Antibody titres were conducted on a fortnightly basis. The results obtained are shown in Table 15.

**Table 14**

| **Contents of the compositions for determination of dependence of immunoenhancing effect of silicone based IC on amounts of antigen and cytokine in Test Example 13** | | | |
|---|---|---|---|
| **Group** | | **Composition (µg)** | |
| | | **IL-1** | **Avidin** |
| 1 | covered rod | 0 | 100 |
| 2 | covered rod | 0 | 10 |
| 3 | covered rod | 0 | 5 |
| 4 | matrix | 0 | 5 |
| 5 | covered rod | 50 | 100 |
| 6 | covered rod | 50 | 10 |
| 7 | covered rod | 25 | 5 |
| 8 | matrix | 50 | 100 |
| 9 | matrix | 50 | 10 |
| 10 | matrix | 25 | 5 |
| 11 | in PBS | 0 | 100 |
| 12 | in PBS | 0 | 10 |
| 13 | in PBS | 0 | 5 |
| 14 | in PBS | 50 | 100 |
| 15 | in PBS | 50 | 10 |
| 16 | in PBS | 25 | 5 |
| 17 | in alum | 0 | 100 |
| 18 | in alum | 0 | 10 |
| 19 | in alum | 0 | 5 |

**Table 15**

| **Dependence of immunoenhancing effect of silicone based lcs on amount of antigen and cytokine in Test Example 13 (50% midpoint titres)** | | | | | | | | |
|---|---|---|---|---|---|---|---|---|
| Type | Gp. | Composition (µg) | | Post-Primary | | | | |
| | | IL-1β | Avidin | 14d | 28d | 42d | 56d | 70d |
| CR | 1 | 0 | 100 | 398 | 501 | 251 | 158 | 158 |
| CR | 2 | 0 | 10 | <50 | 631 | 501 | <50 | <50 |
| CR | 3 | 0 | 5 | 126 | 1259 | 1778 | <50 | <50 |
| M | 4 | 0 | 5 | 794 | 1259 | 251 | 126 | 126 |
| CR | 5 | 50 | 100 | 3981 | 5623 | 2512 | 1995 | 1995 |
| CR | 6 | 50 | 10 | 3981 | 3981 | 794 | 1259 | 1259 |
| CR | 7 | 25 | 5 | 5012 | 5012 | 1778 | 794 | 794 |
| M | 8 | 50 | 100 | 501 | 794 | 196 | 158 | 158 |
| M | 9 | 50 | 10 | 251 | 501 | 316 | 158 | 158 |
| M | 10 | 25 | 5 | 158 | 316 | 316 | 126 | 80 |
| S | 11 | 0 | 100 | 126 | 158 | 80 | <50 | <50 |
| S | 12 | 0 | 10 | <50 | 126 | <50 | <50 | <50 |
| S | 13 | 0 | 5 | <50 | 251 | <50 | <50 | 50 |
| S | 14 | 50 | 100 | 398 | 196 | 251 | 126 | 126 |
| S | 15 | 50 | 10 | 196 | 251 | 196 | <50 | <50 |
| S | 16 | 25 | 5 | 58 | 251 | <50 | <50 | <50 |
| A | 17 | 0 | 100 | 1995 | 1995 | 631 | 631 | 251 |
| A | 18 | 0 | 10 | 1259 | 1995 | 631 | 631 | 251 |
| A | 19 | 0 | 5 | 1000 | 631 | 316 | 316 | 251 |

| | | | | | | | | |
|---|---|---|---|---|---|---|---|---|
| Cl: collagen IC M: Matrix IC A: PBS solution containing alum S: PBS solution CR: covered rod IC | | | | | | | | |

These results demonstrate the high titre and persistence of antibody occurring in response to the covered rod ICs. The most persistent response was elicited in the presence of the highest dose of IL-1β and antigen. At the 70 day time point, covered rod silicone ICs incorporating IL-1β were clearly superior to liquid formulations of avidin (titres approximately 5-fold).

Collagen and silicone IC could effectively act as vaccine vehicles: the immunogenicity of the antigen was retained, and the biological activity of the cytokine adjuvant was preserved.

Collagen IC and silicone matrix IC exhibited inherent adjuvant activity.

When IL-1β was incorporated into the IC at appropriate levels, antibody responses exceeded responses induced by alum adjuvant.

Covered rod silicone IC incorporating IL-1β as adjuvant induced significantly higher antibody responses than any other composition tested (liquid or IC). In addition the antibody response was sustained for longer periods than for other compositions.

No adverse systemic or histological responses have been observed that would exclude the use of the ICs as safe vaccine vehicles.

## Claims

1. An immunopotentiating composition which comprises an antigen or antigen-inducing substance and a carrier, wherein
(a) the carrier is collagen or polydimethylsiloxane and the antigen or antigen-inducing substance is dispersed therein or encapsulated therewithin;
(b) the antigen induces an immune response specific thereto, wherein the immune response is
(i) an antibody production, effective in the prevention and/or treatment of diseases in a human, a mammal other than a human, or a bird,
(ii) effective in the prevention or treatment of a disease,
(iii) effective in the prevention of infection with a virus, mycoplasmata, bacterium or parasite, or
(iv) used for animal production,
or the antigen is a superantigen;
(c) the immunopotentiating composition is in a solid dosage form; and
(d) the antigen-inducing substance is a plasmid or virus with a nucleic acid (gene sequence) coding for an antigen capable of inducing an immune response specific to a member of the group consisting of viruses, mycoplasmata, bacteria, parasites, toxins and tumor cells as incorporated therein so that said antigen can be produced in vivo, or for a superantigen as incorporated therein so that said superantigen can be produced in vivo.

2. The immunopotentiating composition as claimed in claim 1, wherein
(a) the carrier comprises a single carrier material,
(b) the antigen induces an immune response specific thereto, wherein the immune response is
(i) an antibody production, effective in the prevention and/or treatment of diseases in a human, a mammal other than human, or a bird,
(ii) effective in the prevention or treatment of a disease,
(iii) effective in the prevention of infection with a virus, mycoplasmata, bacterium or parasite, or
(iv) used for animal production.

3. The immunopotentiating composition as claimed in claim 1 or 2, wherein the antigen or antigen-inducing substance is capable of inducing an immune response specific to a member of the group consisting of viruses, mycoplasmata, bacteria, parasites, toxins and tumor cells.

4. The immunopotentiating composition as claimed in claim 3, wherein the antigen or antigen-inducing substance is a substance obtained by using the chemical technology, recombinant DNA technology, cell culture technology or fermentation technology.

5. The immunopotentiating composition as claimed in claim 3, wherein the antigen is derived from a member of the group consisting of viruses, mycoplasmata, bacteria, parasites, toxins and tumor cells, by attenuation, detoxification or rendering the same nonpathogenic.

6. The immunopotentiating composition as claimed in claim 3, wherein the antigen or antigen-inducing substance is a substance obtained from a member of the group consisting of viruses, mycoplasmata, bacteria, parasites, toxins and tumor cells.

7. The immunopotentiating composition as claimed in claim 1 or 2, wherein said composition is a film, sponge, rod or bar, or minute particles.

8. The immunopotentiating composition as claimed in claim 1 which further comprises a substance having immunoactivating, immunostimulating or immunomodulating activity.

9. The immunopotentiating composition as claimed in claim 8, wherein the substance having immunoactivating, immunostimulating or immunomodulating activity is a cytokine.

10. The immunopotentiating composition as claimed in claim 8 or 9, wherein
(a) the carrier comprises a single carrier material,
(b) the antigen induces an immune response specific thereto, wherein the immune response is
(i) an antibody production, effective in the prevention and/or treatment of diseases in a human, a mammal other than a human, or a bird,
(ii) effective in the prevention or treatment of a disease,
(iii) effective in the prevention of infection with a virus, mycoplasmata, bacterium or parasite, or
(iv) used for animal production.

11. The immunopotentiating composition as claimed in any of claims 8 to 10 wherein the antigen or antigen-inducing substance is capable of inducing an immune response specific to a member of the group consisting of viruses, mycoplasmata, bacteria, parasites, toxins and tumor cells.

12. The immunopotentiating composition as claimed in claim 11, wherein the antigen or antigen-inducing substance is a substance obtained by using the chemical technology, recombinant DNA technology, cell culture technology or fermentation technology.

13. The immunopotentiating composition as claimed in claim 11, wherein the antigen is derived from a member of the group consisting of viruses, mycoplasmata, bacteria, parasites, toxins and tumor cells by attenuation, detoxification or rendering the same non-pathogenic.

14. The immunopotentiating composition as claimed in claim 11, wherein the antigen or antigen-inducing substance is a substance obtained from a member of the group consisting of viruses, mycoplasmata, bacteria, parasites, toxins and tumor cells.

15. The immunopotentiating composition as claimed in any of claims 8 to 10 wherein said composition is a film, sponge, rod or bar, or minute particles.

16. The immunopotentiating composition as claimed in claim 1, wherein the antigen is a superantigen.

17. The immunopotentiating composition as claimed in claim 8 or 9, wherein the antigen is a superantigen.

18. The immunopotentiating composition as claimed in claim 1 or 8, wherein said composition is of a bar or rod-like shape.

19. The immunopotentiating composition as daimed in claim 18, wherein said composition is of a coated or covered rod-like shape.

20. The immunopotentiating composition as claimed in claim 19, wherein the carrier is a polydimethylsiloxane.

21. The immunopotentiating composition as claimed in claim 18, which, in use, exhibits a sustained release profile.

22. The immunopotentiating composition as claimed in claim 8 wherein the substance having immunoactivating, immunostimulating or immunomodulating activity is an adjuvant selected from the group consisting of chemokines, growth factors, adjuvant peptides and DNA sequences, alum. Freund's complete adjuvant, Freund's incomplete adjuvant, iscom, saponins, hexadecylamine, dimethyldioctadecylammonium bromide, Abridin, cell wall skeletal components, cholera toxin, lipopolysaccharide endotoxins, and liposomes.

23. The immunopotentiating composition as claimed in claim 9, wherein the cytokine is included in the composition in the absence of a solvent.

24. A method of obtaining an antibody which comprises recovering the antibody from blood which has been collected from a mammal other than a human being or a bird to which the immunopotentiating composition of any of claims 1 to 23 has been administered, thereby modulating the immune response in said mammal or bird.

## Patentansprüche

1. Immunpotenzierende Zusammensetzung, welche ein Antigen oder einen antigeninduzierenden Stoff und einen Träger umfasst, wobei
(a) der Träger Kollagen oder Polydimethylsiloxan ist und das Antigen oder der antigeninduzierende Stoff darin dispergiert oder damit eingekapselt ist;
(b) das Antigen eine dazu spezifische Immunantwort einleitet, wobei die Immunantwort
(i) eine Antikörperproduktion ist, die wirksam ist zur Prävention und/oder Behandlung von Erkrankungen im Menschen, einem nicht-menschlichen Säuger oder einem Vogel,
(ii) wirksam zur Prävention oder Behandlung einer Erkrankung ist,
(iii) wirksam zur Prävention einer Infektion mit einem Virus, Mycoplasmen, Bakterien oder Parasiten ist, oder
(iv) in der Tierproduktion verwendet wird,
oder das Antigen ein Superantigen ist;
(c) die immunpotenzierende Zusammensetzung in einer festen Dosierungsform ist, und
(d) der antigeninduzirende Stoff ein Plasmid oder ein Virus mit einer darin enthaltenen Nucleinsäure (Gensequenz) ist, die ein darin enthaltenes Antigen codiert, das imstande ist, eine spezifischen Immunantwort gegen ein Mitglied aus der Gruppe bestehend aus Viren, Mycoplasmen, Bakterien, Parasiten, Toxinen und Tumorzellen zu induzieren, so dass das Antigen in vivo produziert werden kann oder ein darin enthaltenes Superantigen codiert, so dass das Superantigen in vivo produziert werden kann.

2. Immunpotenzierende Zusammensetzung nach Anspruch 1, wobei
(a) der Träger ein einzelnes Trägermaterial umfasst;
(b) das Antigen eine dazu spezifische Immunantwort induziert, wobei die Immunantwort
(i) eine Antikörperproduktion ist, die wirksam ist zur Prävention und/oder Behandlung von Erkrankungen im Menschen, einem nicht-menschlichen Säuger oder einem Vogel,
(ii) wirksam zur Prävention oder Behandlung einer Erkrankung ist,
(iii) wirksam zur Prävention einer Infektion mit einem Virus, Mycoplasmen, Bakterien oder Parasiten ist, oder
(iv) in der Tierproduktion verwendet wird.

3. Immunpotenzierende Zusammensetzung nach Anspruch 1 oder 2, wobei das Antigen oder der antigeninduzierende Stoff imstande ist, eine spezifische Immunantwort gegen ein Mitglied aus der Gruppe bestehend aus Viren, Mycoplasmen, Bakterien, Parasiten, Toxinen und Tumorzellen zu induzieren.

4. Immunpotenzierende Zusammensetzung nach Anspruch 3, wobei das Antigen oder der antigenerzeugende Stoff ein durch chemische Technologie, rekombinante DNA-Technologie, Zellkulturtechnologie oder Fermentationstechnologie erhaltener Stoff ist.

5. Immunpotenzierende Zusammensetzung nach Anspruch 3, wobei das Antigen abgeleitet ist von einem Mitglied aus der Gruppe bestehend aus Viren, Mycoplasmen, Bakterien, Parasiten, Toxinen und Tumorzellen mittels Abschwächung, Detoxifikation oder deren Umwandlung in eine nicht-pathogene Form.

6. Immunpotenzierende Zusammensetzung nach Anspruch 3, wobei das Antigen oder der antigeninduzierende Stoff ein von einem Mitglied aus der Gruppe bestehend aus Viren, Mycoplasmen, Bakterien, Parasiten, Toxinen und Tumorzellen erhaltenen Stoff ist.

7. Immunpotenzierende Zusammensetzung nach Anspruch 1 oder 2, wobei die Zusammensetzung ein Film, Schwamm, Stange oder Stab oder winzige Partikel ist.

8. Immunpotenzierende Zusammensetzung nach Anspruch 1, welche ferner einen Stoff mit immunaktivierender, immunstimulierender oder immunmodulierender Aktivität umfasst.

9. Immunpotenzierende Zusammensetzung nach Anspruch 8, wobei der Stoff mit immunaktivierender, immunstimulierender oder immunmodulierender Aktivität ein Cytokin ist.

10. Immunpotenzierende Zusammensetzung nach Anspruch 8 oder 9, wobei
(a) der Träger ein einzelnes Trägermaterial umfasst;
(b) das Antigen eine dazu spezifische Immunantwort induziert, wobei die Immunantwort
(i) eine Antikörperproduktion ist, die wirksam ist zur Prävention und/oder Behandlung von Erkrankungen im Menschen, einem nicht-menschlichen Säuger oder einem Vogel,
(ii) wirksam zur Prävention oder Behandlung einer Erkrankung ist,
(iii) wirksam zur Prävention einer Infektion mit einem Virus, Mycoplasmen, Bakterien oder Parasiten ist, oder
(iv) in der Tierproduktion verwendet wird.

11. Immunpotenzierende Zusammensetzung nach einem der Ansprüche 8 bis 10, wobei das Antigen oder der antigeninduzierende Stoff im Stande ist, eine spezifische Immunantwort gegen ein Mitglied aus der Gruppe bestehend aus Viren, Mycoplasmen, Bakterien, Parasiten, Toxinen und Tumorzellen zu induzieren.

12. Immunpotenzierende Zusammensetzung nach Anspruch 11, wobei das Antigen oder der antigeninduzierende Stoff ein durch chemische Technologie, rekombinante DNA-Technologie, Zellkulturtechnologie oder Fermentationstechnologie erhaltener Stoff ist.

13. Immunpotenzierende Zusammensetzung nach Anspruch 11, wobei das Antigen abgeleitet ist von einem Mitglied aus der Gruppe bestehend aus Viren, Mycoplasmen, Bakterien, Parasiten, Toxinen und Tumorzellen mittels Abschwächung, Detoxifikation oder deren Umwandlung in eine nicht-pathogene Form.

14. Immunpotenzierende Zusammensetzung nach Anspruch 11, wobei das Antigen oder der antigeninduzierende Stoff ein aus einem Mitglied aus der Gruppe bestehend aus Viren, Mycoplasmen, Bakterien, Parasiten, Toxinen und Tumorzellen erhaltener Stoff ist.

15. Immunpotenzierende Zusammensetzung nach einem der Ansprüche 8 bis 10, wobei die Zusammensetzung ein Film, Schwamm, Stange oder Stab oder winzige Partikel ist.

16. Immunpotenzierende Zusammensetzung nach Anspruch 1, wobei das Antigen ein Superantigen ist.

17. Immunpotenzierende Zusammensetzung nach Anspruch 8 oder 9, wobei das Antigen ein Superantigen ist.

18. Immunpotenzierende Zusammensetzung nach Anspruch 1 oder 8, wobei die Zusammensetzung eine Stangen- oder Stabform hat.

19. Immunpotenzierende Zusammensetzung nach Anspruch 18, wobei die Zusammensetzung in einer beschichteten oder ummantelten Stabform vorliegt.

20. Immunpotenzierende Zusammensetzung nach Anspruch 19, wobei der Träger Polydimethylsiloxan ist.

21. Immunpotenzierende Zusammensetzung nach Anspruch 18, welche bei Verwendung ein Profil mit verzögerter Freisetzung zeigt.

22. Immunpotenzierende Zusammensetzung nach Anspruch 8, wobei der Stoff mit immunaktivierender, immunstimulierender oder immunmodulierender Aktivität ein Adjuvans ist, ausgewählt aus der Gruppe bestehend aus Chemokinen, Wachstumsfaktoren, Adjuvanspeptiden und DNA-Sequenzen, Alaun, komplettem Freundschem-Adjuvans, inkomplettem Freundschen-Adjuvans, Iscom, Saponinen, Hexadecylamin, Dimethyldioctadecylamonium-bromid, Abridin, Zellwandskelettkomponenten, Choleratoxin, Lipopolysaccharidendotoxinen, und Liposomen.

23. Immunpotenzierende Zusammensetzung nach Anspruch 9, wobei das Cytokin in der Zusammensetzung in Abwesenheit eines Lösungsmittels enthalten ist.

24. Verfahren zum Erhalt eines Antikörpers umfassend das Gewinnen des Antikörpers aus dem von einem nicht-menschlichen Säuger oder von einem Vogel enthaltenes Blut, denen die immunpotenzierende Zusammensetzung nach einem der Ansprüche 1 bis 23 verabreicht wurde, wobei die Immunantwort des Säugers oder des Vogels moduliert wurde.

## Revendications

1. Composition immunostimulante qui comprend un antigène ou une substance induisant un antigène et un support, dans laquelle
(a) le support est du collagène ou du polydiméthylsiloxane et l'antigène ou la substance induisant un antigène est dispersé dans le support ou encapsulé par le support ;
(b) l'antigène induit une réponse immunitaire spécifique, la réponse immunitaire étant
(i) une production d'anticorps, efficace dans la prévention et/ou le traitement de maladies chez un homme, un mammifère autre qu'un homme ou un oiseau,
(ii) efficace dans la prévention ou le traitement d'une maladie,
(iii) efficace dans la prévention d'une infection par un virus, un mycoplasme, une bactérie ou un parasite, ou
(iv) utilisée pour la production d'un animal,
ou l'antigène est un superantigène ;
(c) la composition immunostimulante est une forme d'administration solide ; et
(d) la substance induisant l'antigène est un plasmide ou un virus contenant un acide nucléique (séquence de gène) codant pour un antigène capable d'induire une réponse immunitaire spécifique d'un élément du groupe constitué par les virus, les mycoplasmes, les bactéries, les parasites, les toxines et les cellules tumorales, incorporé de telle façon que ledit antigène puisse être produit *in vivo* ou codant pour un superantigène incorporé de telle façon que ledit superantigène puisse être produit *in vivo.*

2. Composition immunostimulante selon la revendication 1, dans laquelle
(a) le support comprend une seule matière support ;
(b) l'antigène induit une réponse immunitaire spécifique, la réponse immunitaire étant
(i) une production d'anticorps, efficace dans la prévention et/ou le traitement de maladies chez un homme, un mammifère autre qu'un homme ou un oiseau,
(ii) efficace dans la prévention ou le traitement d'une maladie,
(iii) efficace dans la prévention d'une infection par un virus, un mycoplasme, une bactérie ou un parasite, ou
(iv) utilisée pour la production d'un animal.

3. Composition immunostimulante selon la revendication 1 ou 2, dans laquelle l'antigène ou la substance induisant l'antigène est capable d'induire une réponse immunitaire spécifique d'un élément du groupe constitué par les virus, les mycoplasmes, les bactéries, les parasites, les toxines et les cellules tumorales.

4. Composition immunostimulante selon la revendication 3, dans laquelle l'antigène ou la substance induisant l'antigène est une substance obtenue en utilisant une technologie chimique, une technologie d'ADN recombiné, une technologie de culture cellulaire ou une technologie de fermentation.

5. Composition immunostimulante selon la revendication 3, dans laquelle l'antigène est dérivé d'un élément du groupe constitué par les virus, les mycoplasmes, les bactéries, les parasites, les toxines et les cellules tumorales, par atténuation, par détoxification ou en rendant l'élément non-pathogène.

6. Composition immunostimulante selon la revendication 3, dans laquelle l'antigène ou la substance induisant un antigène est une substance obtenue à partir d'un élément du groupe constitué par les virus, les mycoplasmes, les bactéries, les parasites, les toxines et les cellules tumorales.

7. Composition immunostimulante selon la revendication 1 ou 2, dans laquelle ladite composition est un film, une éponge, un bâtonnet ou une barrette, ou de très petites particules.

8. Composition immunostimulante selon la revendication 1, qui comprend en outre une substance ayant une activité d'immunoactivation, d'immunostimulation ou d'immunomodulation.

9. Composition immunostimulante selon la revendication 8, dans laquelle la substance ayant une activité d'immunoactivation, d'immunostimulation ou d'immunomodulation est une cytokine.

10. Composition immunostimulante selon la revendication 8 ou 9, dans laquelle
(a) le support comprend une seule matière support ;
(b) l'antigène induit une réponse immunitaire spécifique, la réponse immunitaire étant
(i) une production d'anticorps, efficace dans la prévention et/ou le traitement de maladies chez un homme, un mammifère autre qu'un homme ou un oiseau,
(ii) efficace dans la prévention ou le traitement d'une maladie,
(iii) efficace dans la prévention d'une infection par un virus, un mycoplasme, une bactérie ou un parasite, ou
(iv) utilisée pour la production d'un animal.

11. Composition immunostimulante selon l'une quelconque des revendications 8 à 10, dans laquelle l'antigène ou la substance induisant l'antigène est capable d'induire une réponse immunitaire spécifique d'un élément du groupe constitué par les virus, les mycoplasmes, les bactéries, les parasites, les toxines et les cellules tumorales.

12. Composition immunostimulante selon la revendication 11, dans laquelle l'antigène ou la substance induisant l'antigène est une substance obtenue en utilisant une technologie chimique, une technologie d'ADN recombinée, une technologie de culture cellulaire ou une technologie de fermentation.

13. Composition immunostimulante selon la revendication 11, dans laquelle l'antigène est dérivé d'un élément du groupe constitué par les virus, les mycoplasmes, les bactéries, les parasites, les toxines et les cellules tumorales, par atténuation, par détoxification ou en rendant l'élément non-pathogène.

14. Composition immunostimulante selon la revendication 11, dans laquelle l'antigène ou la substance induisant un antigène est une substance obtenue à partir d'un élément du groupe constitué par les virus, les mycoplasmes, les bactéries, les parasites, les toxines et les cellules tumorales.

15. Composition immunostimulante selon l'une quelconque des revendications 8 à 10, où ladite composition est un film, une éponge, un bâtonnet ou une barrette, ou de très petites particules.

16. Composition immunostimulante selon la revendication 1, dans laquelle l'antigène est un superantigène.

17. Composition immunostimulante selon la revendication 8 ou 9, dans laquelle l'antigène est un superantigène.

18. Composition immunostimulante selon la revendication 1 ou 8, où ladite composition a la forme d'une barrette ou d'un bâtonnet.

19. Composition immunostimulante selon la revendication 18, où ladite composition a la forme d'un bâtonnet enrobé ou recouvert.

20. Composition immunostimulante selon la revendication 19, où le support est un polydiméthylsiloxane.

21. Composition immunostimulante selon la revendication 18, qui, en utilisation, présente un profil de libération prolongée.

22. Composition immunostimulante selon la revendication 8, dans laquelle la substance ayant une activité d'immunoactivation, d'immunostimulation ou d'immunomodulation est un adjuvant choisi dans le groupe constitué par les chimiokines, les facteurs de croissance, les peptides adjuvants et les séquences d'ADN, l'alun, l'adjuvant complet de Freund, l'adjuvant incomplet de Freund, l'iscom, les saponines, l'hexadécylamine, le bromure de diméthyldioctadécylammonium, l'Abridine, les composants du squelette de la paroi cellulaire, la toxine du choléra, les endotoxines lipopolysaccharidiques et les liposomes.

23. Composition immunostimulante selon la revendication 9, où la cytokine est incluse dans la composition en l'absence d'un solvant.

24. Méthode d'obtention d'un anticorps qui comprend la récupération de l'anticorps à partir d'un sang prélevé chez un mammifère autre qu'un être humain ou chez un oiseau auquel la composition immunostimulante selon l'une quelconque des revendications 1 à 23 a été administrée, modulant ainsi la réponse immunitaire chez ledit mammifère ou oiseau.
